(19) Europäisches Patentamt
European Patent Office
Office européen des brevets

(11) EP 0 799 828 A2

(12) **EUROPÄISCHE PATENTANMELDUNG**

(43) Veröffentlichungstag: **08.10.1997 Patentblatt 1997/41**

(21) Anmeldenummer: **97104940.8**

(22) Anmeldetag: **24.03.1997**

(51) Int. Cl.[6]: **C07D 487/04**, A61K 31/505 // (C07D487/04, 239:00, 209:00)

(84) Benannte Vertragsstaaten:
**AT BE CH DE DK ES FI FR GB GR IE IT LI LU MC NL PT SE**
Benannte Erstreckungsstaaten:
**LT LV RO SI**

(30) Priorität: **04.04.1996 DE 19613550**

(71) Anmelder: **BAYER AG**
**51368 Leverkusen (DE)**

(72) Erfinder:
- **Müller, Ulrich, Dr.**
  **42111 Wuppertal (DE)**
- **Eckenberg, Peter, Dr.**
  **40699 Erkrath (DE)**
- **Grützmann, Rudi, Dr.**
  **42657 Solingen (DE)**
- **Bischoff, Hilmar, Dr.**
  **42113 Wuppertal (DE)**
- **Denzer, Dirk, Dr.**
  **42115 Wuppertal (DE)**
- **Wohlfeil, Stefan, Dr.**
  **40721 Hilden (DE)**
- **Lohmer, Stefan, Dr.**
  **20133 Milano (IT)**
- **Nielsch, Ulrich, Dr.**
  **42113 Wuppertal (DE)**
- **Kolkhof, Peter, Dr.**
  **42115 Wuppertal (DE)**

(54) **Neue Pyrimido [1,2-a] indole**

(57) Die erfindungsgemäßen Pyrimido[1,2-a]indole werden hergestellt durch Umsetzung von entsprechend substituierten Phenylessigsäurederivaten mit Phenylglycinolen. Die Pyrimido[1,2-a]indole können als Wirkstoffe in Arzneimittel verwendet werden, insbesondere in Arzneimittel mit antiatherosklerotischer Wirkung.

EP 0 799 828 A2

**Beschreibung**

Die vorliegende Erfindung betrifft Pyrimido[1,2-a]indole, Verfahren zu ihrer Herstellung und ihre Verwendung als Arzneimittel, insbesondere als antiatherosklerotische Arzneimittel.

Es ist bekannt, daß erhöhte Blutspiegel von Triglyceriden (Hypertriglyceridämie) und Cholesterin (Hypercholesterinämie) mit der Genese von atherosklerotischen Gefäßwandveränderungen und koronaren Herzkrankheiten assoziiert sind.

Ein deutlich erhöhtes Risiko für die Entwicklung koronarer Herzerkrankungen liegt darüberhinaus vor, wenn diese beiden Risikofaktoren kombiniert auftreten, was wiederum mit einer Überproduktion an Apolipoprotein B-100 einhergeht. Es ist daher nach wie vor ein starkes Bedürfnis, wirksame Arzneimittel zur Bekämpfung der Atherosklerose sowie koronarer Herzkrankheiten zur Verfügung zu stellen.

Die vorliegende Erfindung betrifft Pyrimido[1,2-a]indole der allgemeinen Formel (I)

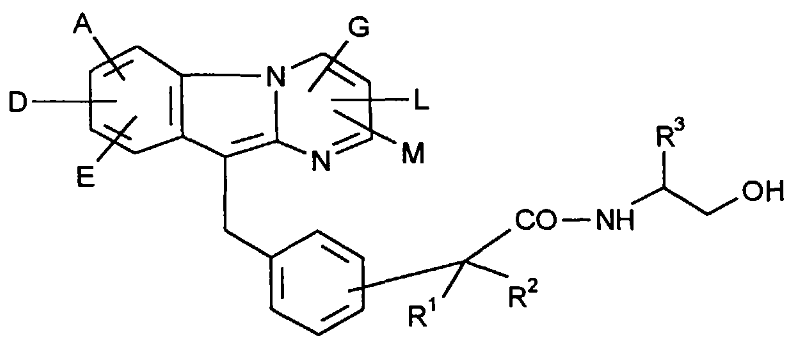

(I)

in welcher

A, D, E, G, L und M gleich oder verschieden sind und für Wasserstoff Halogen, Trifluormethyl, Carboxy, Hydroxy, geradkettiges oder verzweigtes Alkoxy oder Alkoxycarbonyl mit jeweils bis zu 6 Kohlenstoffatomen oder geradkettiges oder verzweigtes Alkyl mit bis zu 6 Kohlenstoffatomen stehen, das seinerseits durch Hydroxy oder durch geradkettiges oder verzweigtes Alkoxy mit bis zu 4 Kohlenstoffatomen substituiert sein kann,

$R^1$ und $R^2$ gleich oder verschieden sind und für Wasserstoff, Cycloalkyl mit 3 bis 8 Kohlenstoffatomen oder geradkettiges oder verzweigtes Alkyl mit bis zu 10 Kohlenstoffatomen stehen, das gegebenenfalls durch Cycloalkyl mit 3 bis 6 Kohlenstoffatomen substituiert ist, oder für Phenyl stehen, das gegebenenfalls durch Halogen oder Trifluormethyl substituiert ist, oder

$R^1$ und $R^2$ gemeinsam mit dem Kohlenstoffatom einen 4-8 gliedrigen Cycloalkylring bilden

und

$R^3$ für Phenyl steht, das gegebenenfalls bis zu 3-fach gleich oder verschieden durch Nitro, Carboxy, Halogen, Cyano oder durch geradkettiges oder verzweigtes Alkenyl oder Alkoxycarbonyl mit jeweils bis zu 6 kohlenstoffatomen oder durch geradkettiges oder verzweigtes Alkyl mit bis zu 6 Kohlenstoffatomen substituiert ist, das gegebenenfalls durch Hydroxy, Carboxy oder durch geradkettiges oder verzweigtes Alkoxy oder Alkoxycarbonyl mit jeweils bis zu 6 Kohlenstoffatomen substituiert ist, und/oder gegebenenfalls durch eine Gruppe der Formel $-OR^4$ oder $-NR^5R^6$ substituiert ist, worin

$R^4$ Wasserstoff oder geradkettiges oder verzweigtes Alkyl oder Alkenyl mit jeweils bis zu 6 Kohlenstoffatomen bedeutet,

$R^5$ bzw. $R^6$ gleich oder verschieden sind und Phenyl, Wasserstoff oder geradkettiges oder verzweigtes Alkyl mit bis zu 6 Kohlenstoffatomen bedeuten, oder geradkettiges oder verzweigtes Acyl mit bis zu 8 Kohlenstoffatomen bedeuten, das gegebenenfalls durch eine Gruppe der Formel $-NR^7R^8$ substituiert ist, worin

$R^7$ und $R^8$ gleich oder verschieden sind und Wasserstoff oder geradkettiges oder verzweigtes Acyl mit bis zu 8 Kohlenstoffatomen bedeuten,

gegebenenfalls in einer isomeren Form und deren Salze.

Die erfindungsgemäßen Pyrimido[1,2-a]indole können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomeren und Diastereomeren lassen sich in bekannter Weise in die stereoisomer einheitlichen Bestandteile trennen.

Bevorzugt sind Verbindungen der allgemeinen Formel (I),
in welcher

A, D, E, G, L und M gleich oder verschieden sind und
für Wasserstoff, Fluor, Chlor, Brom, Trifluormethyl, Carboxy, Hydroxy, geradkettiges oder verzweigtes Alkoxy oder Alkoxycarbonyl mit jeweils bis zu 4 Kohlenstoffatomen oder geradkettiges oder verzweigtes Alkyl mit bis zu 4 Kohlenstoffatomen stehen, das seinerseits durch Hydroxy oder durch geradkettiges oder verzweigtes Alkoxy mit bis zu 3 Kohlenstoffatomen substituiert sein kann,

$R^1$ und $R^2$ gleich oder verschieden sind und
für Wasserstoff, Cyclopropyl, Cyclobutyl, Cyclopentyl, Cyclohexyl, Cycloheptyl, Cyclooctyl oder für geradkettiges oder verzweigtes Alkyl mit bis zu 8 Kohlenstoffatomen stehen, das gegebenenfalls durch Cyclopropyl, Cyclopentyl oder Cyclohexyl substituiert ist, oder für Phenyl stehen, das gegebenenfalls durch Fluor, Chlor oder Brom substituiert ist, oder

$R^1$ und $R^2$ gemeinsam mit dem Kohlenstoffatom einen 4-7 gliedrigen Cycloalkylring bilden

und

$R^3$ für Phenyl steht, das gegebenenfalls bis zu 3-fach gleich oder verschieden durch Nitro, Carboxy, Fluor, Chlor, Brom, Cyano, durch geradkettiges oder verzweigtes Alkenyl oder Alkoxycarbonyl mit jeweils bis zu 4 Kohlenstoffatomen oder durch geradkettiges oder verzweigtes Alkyl mit bis zu 5 Kohlenstoffatomen substituiert ist, das gegebenenfalls durch Hydroxy, Carboxy oder durch geradkettiges oder verzweigtes Alkoxy oder Alkoxycarbonyl mit jeweils bis zu 5 Kohlenstoffatomen substituiert ist, und/oder gegebenenfalls durch eine Gruppe der Formel $-OR^4$ oder $-NR^5R^6$ substituiert ist,
worin

$R^4$ Wasserstoff oder geradkettiges oder verzweigtes Alkyl oder Alkenyl mit jeweils bis zu 4 Kohlenstoffatomen bedeutet,

$R^5$ und $R^6$ gleich oder verschieden sind und
Phenyl, Wasserstoff oder geradkettiges oder verzweigtes Alkyl mit bis zu 5 Kohlenstoffatomen bedeuten, oder geradkettiges oder verzweigtes Acyl mit bis zu 6 Kohlenstoffatomen bedeuten, das gegebenenfalls durch eine Gruppe der Formel $-NR^7R^8$ substituiert ist,
worin

$R^7$ und $R^8$ gleich oder verschieden sind und Wasserstoff oder geradkettiges oder verzweigtes Acyl mit bis zu 6 Kohlenstoffatomen bedeuten,

gegebenenfalls in einer isomeren Form und deren Salze.

Besonders bevorzugt sind Verbindungen der allgemeinen Formel (I), in welcher

A, D, E, G, L und M gleich oder verschieden sind und für Wasserstoff, Fluor, Chlor, Brom, Trifluormethyl, Carboxy, Hydroxy, geradkettiges oder verzweigtes Alkoxy oder Alkoxycarbonyl mit jeweils bis zu 3 Kohlenstoffatomen oder für geradkettiges oder verzweigtes Alkyl mit bis zu 3 Kohlenstoffatomen stehen,

$R^1$ und $R^2$ gleich oder verschieden sind und für Wasserstoff, Cyclopropyl, Cyclobutyl, Cyclopentyl, Cyclohexyl, Cycloheptyl, Cyclooctyl oder für geradkettiges oder verzweigtes Alkyl mit bis zu 6 Kohlenstoffatomen stehen, das gegebenenfalls durch Cyclopentyl oder Cyclohexyl substituiert ist, oder für Phenyl stehen, das gegebenenfalls durch Fluor, Chlor oder Brom substituiert ist, oder

$R^1$ und $R^2$ gemeinsam mit dem Kohlenstoffatom einen 5-7 gliedrigen Cycloalkylring bilden

und

$R^3$ für Phenyl steht, das gegebenenfalls bis zu 3-fach gleich oder verschieden durch Hydroxy, Trifluormethyl, Trifluormethoxy, Carboxy, oder durch geradkettiges oder verzweigtes Alkoxy, Alkyl oder Alkoxycarbonyl mit jeweils bis zu 3 Kohlenstoffatomen substituiert ist,

gegebenenfalls in einer isomeren Form und deren Salze.

Außerdem wurde ein Verfahren zur Herstellung der erfindungsgemäßen Verbindungen der allgemeinen Formel (I), gefunden, dadurch gekennzeichnet, daß man racemische oder auch bereits enantiomerenreine Carbonsäuren oder deren aktivierte Derivate der allgemeinen Formel (II)

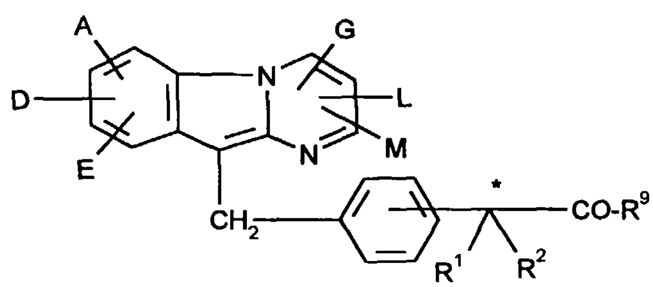

(II)

racemisch oder enantiomerenrein

in welcher

A, D, E, G, L, M, $R^1$ und $R^2$ die angegebene Bedeutung haben

und

$R^9$ für Hydroxy oder für einen aktivierenden Rest, vorzugsweise Chlor steht,

mit Phenylglycinolen der allgemeinen Formel (III)

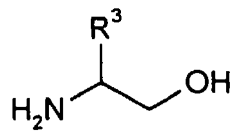 (III)

in welcher

$R^3$ die angegebene Bedeutung hat,

in inerten Lösemitteln, gegebenenfalls in Anwesenheit von Basen und/oder Hilfsstoffen amidiert.

Das erfindungsgemäße Verfahren kann durch folgendes Formelschema beispielhaft erläutert werden:

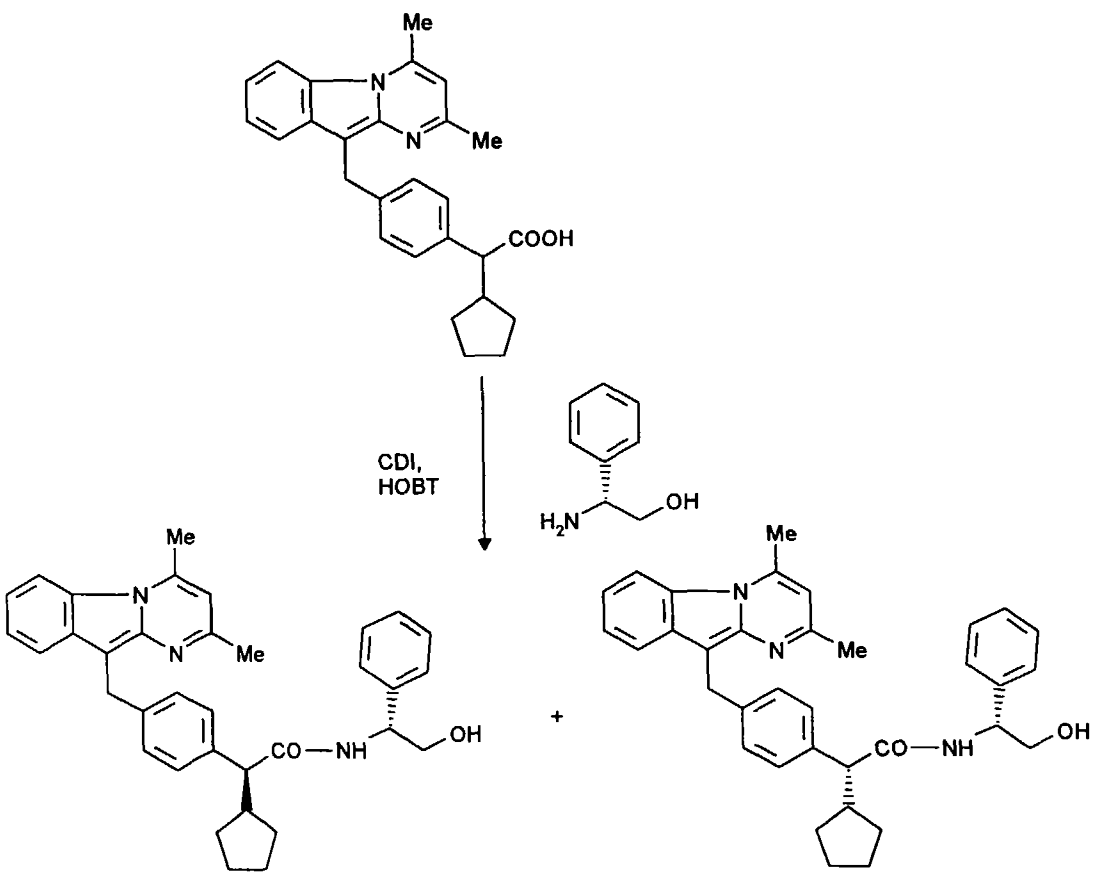


Als Lösemittel für die Amidierung eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Dichlormethan, Tetrahydrofuran, Aceton oder Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Dimethylaminopyridin, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat und Triethylamin.

Die Base wird in einer Menge von 1 mol bis 5 mol, bevorzugt von 1 mol bis 3 mol, bezogen auf 1 mol der Verbindung der allgemeinen Formel (II) eingesetzt.

Die Reaktion wird im allgemeinen in einem Temperaturbereich von 0°C bis 150°C, bevorzugt von +20°C bis +110°C durchgeführt.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die Umsetzung kann gegebenenfalls auch über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die oben aufgeführten Basen können auch als säurebindende Hilfsmittel für die Amidierung eingesetzt werden.

Als Hilfsstoffe eignen sich ebenso Dehydratisierungsreagenzien. Dazu gehören beispielsweise Carbodiimide wie Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Iso-butylchloroformat oder Benzotriazolyloxy-tris-(dimethylamino)phosphoniumhexa-fluorophosphat oder Phosphorsäurediphenyl-esteramid oder Methan-sulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die Hilfsstoffe werden im allgemeinen in einer Menge von 0,5 bis 3 mol, bevorzugt von 1 bis 1,5 mol, bezogen auf 1 mol der entsprechenden Carbonsäuren, eingesetzt.

Die Carbonsäuren der allgemeinen Formel (II) sind neu und können hergestellt werden, indem man zunächst durch Umsetzung von Verbindungen der allgemeinen Formel (IV)

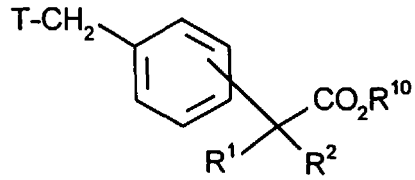

(IV)

in welcher

$R^1$ und $R^2$ die angegebene Bedeutung haben,

T für eine typische Abgangsgruppe wie beispielsweise Chlor, Brom, Jod, Tosylat oder Mesylat, vorzugsweise für Brom steht,

und

$R^{10}$ für $(C_1-C_4)$-Alkyl steht,

mit Verbindungen der allgemeinen Formel (V)

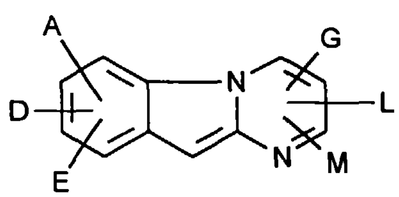

(V)

in welcher

A, D, E, G, L und M die angegebene Bedeutung haben,

die Verbindungen der allgemeinen Formel (VI)

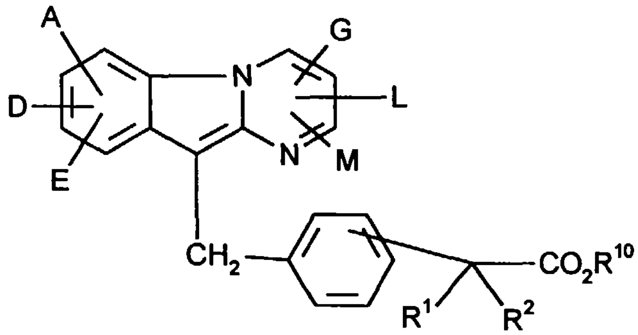

(VI)

in welcher

A, D, E, G, L, M, $R^1$, $R^2$ und $R^{10}$ die oben angegebene Bedeutung haben,

in inerten Lösemitteln, gegebenenfalls in Anwesenheit von Basen herstellt und anschließend die Ester nach üblichen Methoden verseift.

Die enantiomerenreinen Säuren, d.h. Verbindungen der Formel (II), bei denen $R^1$ und $R^2$ verschieden sein müssen, erhält man darüber hinaus in dem man von den D- oder L-Menthyl-Estern der allgemeinen Formel (VII)

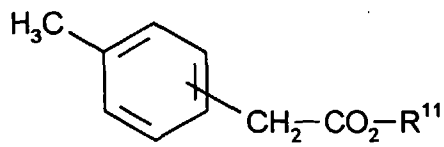

(VII)

in welcher

$R^{11}$ für D- oder L-Menthyl steht,

durch Umsetzung mit Verbindungen der allgemeinen Formel (VIIIa) und (VIIIb)

$R^1$-Z (VIIIa)

$R^2$-Z (VIIIb)

in welcher

$R^1$ bzw. $R^2$ verschieden sind und ansonsten die angegebene Bedeutung haben,

und

Z für Halogen, vorzugsweise Brom steht,

die enantiomerenreinen Menthyl-Ester der allgemeinen Formel (IXa) und (IXb)

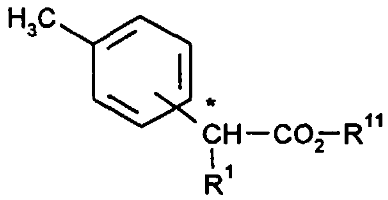 (IXa) 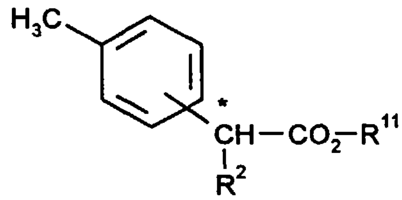 (IXb)

in welcher

$R^1$, $R^2$ und $R^{11}$ die angegebene Bedeutung haben,

herstellt,
diese in einem nächsten Schritt durch eine Halogenierung in die Verbindungen der allgemeinen Formel (Xa) und (Xb)

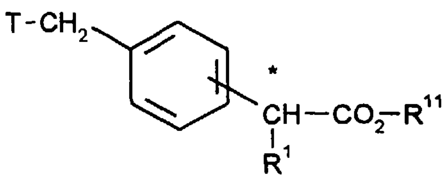 (Xa) 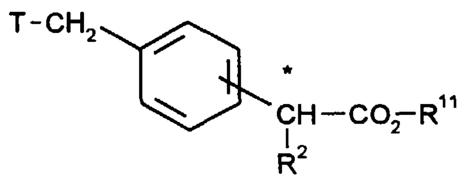 (Xb)

in welcher

$R^1$, $R^2$, T und $R^{11}$ die angegebene Bedeutung haben

überführt,
anschließend durch Umsetzung mit den Verbindungen der allgemeinen Formel (V) die enantiomerenreinen Verbindungen der allgemeinen Formel (XIa) und (XIb)

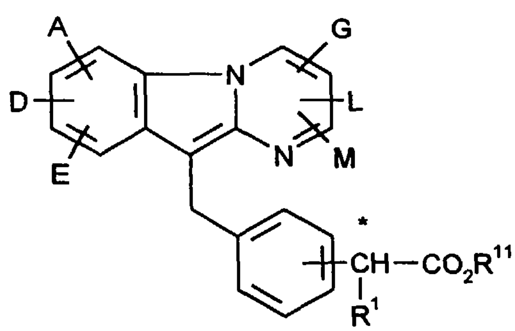

(XIa)

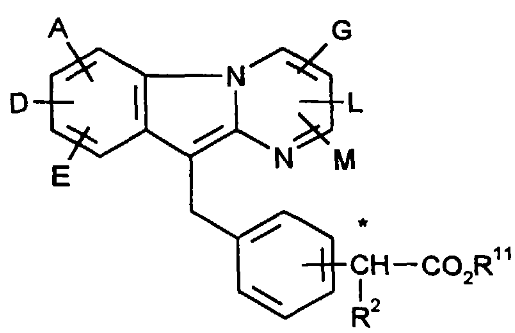

(XIb)

in welcher

A, D, E, G, L, M, $R^1$, $R^2$ und $R^{11}$ die angegebene Bedeutung haben,

herstellt,
und diese dann durch Hydrolyse in die enantiomerenreinen Säuren der allgemeinen Formel (IIa) und (IIb) überführt.

Außerdem können die enantiomerenreinen Säuren der Formel (II) hergestellt werden, indem man zunächst racemische Carbonsäuren der allgemeinen Formel (XII)

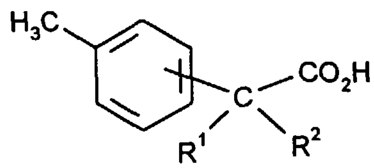 (XII)

durch Umsetzung mit (R)- oder (S)-Phenylethylamin in inerten Lösemitteln und anschließender Kristallisation der Phenethylammoniumsalze und anschließende Hydrolyse der Salze in die enantiomerenreinen Verbindungen der allgemeinen Formel (XIIIa,b)

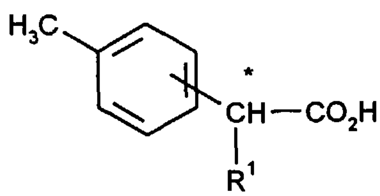 (XIIIa)

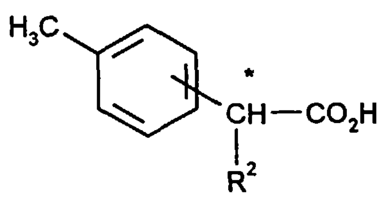 (XIIIb)

überführt,
diesen in einem weiteren Schritt mit Isobuten, in inerten Lösemitteln und in Anwesenheit von Säuren die enantiomerenreinen Ester der allgemeinen Formel (XIVa,b)

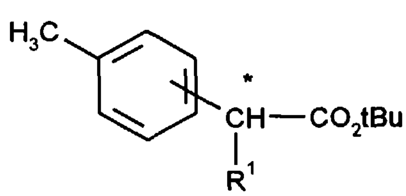 (XIVa)

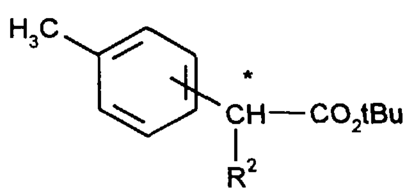 (XIVb)

herstellt,

wie oben beschrieben durch eine Halogenierung in die enantiomerenreinen Verbindungen der allgemeinen Formel (XVa,b)

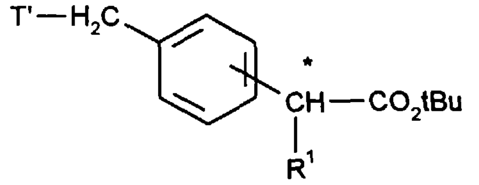

(XVa)

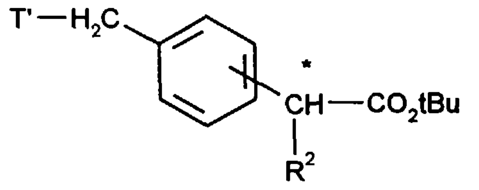

(XVb)

überführt,
und durch Umsetzung mit den Verbindungen der allgemeinen Formel (V) in die enantiomerenreinen Ester der allgemeinen Formel (XVIa,b)

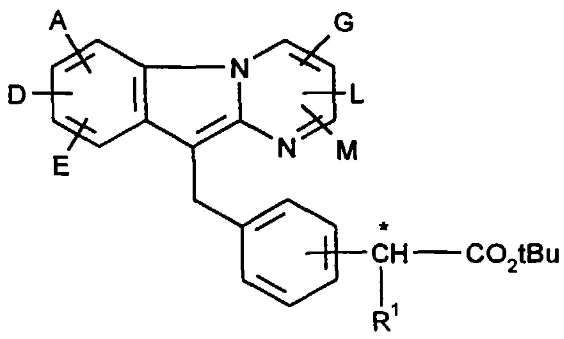

(XVIa)

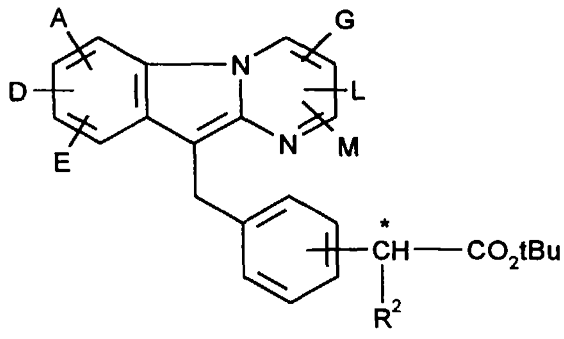

(XVIb)

überführt,
und in den letzten Schritten, wie vorne beschrieben, die entsprechenden enantiomerenreinen Säuren und aktivierten Derivate herstellt.

Als Lösemittel für die Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind

Dimethylformamid, Toluol und Tetrahydrofuran.

Als Basen für die erfindungsgemäßen Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate und -hydrogencarbonate wie Natriumcarbonat, Natriumhydrogencarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Dimethylaminopyridin, Methylpiperidin oder Morpholin. Es ist auch möglich als Basen Alkalimetalle wie Natrium oder deren Hydride wie Natriumhydrid einzusetzen. Bevorzugt sind Natriumhydrogencarbonat, Kaliumcarbonat und Kalium-tert.butylat, DBU oder DABCO.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man äquimolare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Das erfindungsgemäß allgemeine Verfahren [A] wird im allgemeinen in einem Temperaturbereich von -30°C bis +150°C, bevorzugt von 80°C bis 150°C, durchgeführt.

Für die einzelnen Schritte zur Herstellung enantiomeren reiner Säuren eignen sich vorzugsweise folgende Bedingungen:

Die Herstellung der Verbindungen der allgemeinen Formel (IX) erfolgt vorzugsweise in Dimethylformamid und Kalium-tert.butanolat in einem Temperaturbereich von -10°C bis +10°C.

Die Halogenierung der Verbindungen der allgemeinen Formel (X) wird in Chlorbenzol mit 1,3-Dibrom-5,5-dimethylhydantoin in Anwesenheit von Azobisisobutyronitril in einem Temperaturbereich von 0°C bis 110°C durchgeführt.

Die Umsetzung zu den Verbindungen der allgemeinen Formel (XI) erfolgt unter Schutzgasatmosphäre in Dimethylformamid und Kalium-tert.butanolat in einem Temperaturbereich von 0°C bis 30°C.

Die Verseifung der Verbindungen der allgemeinen Formel (XI) kann wie oben beschrieben durchgeführt werden, wobei das System HBr/Ameisensäure besonders bevorzugt ist. Die Verseifung wird in einem Temperaturbereich von 20°C bis 100°C durchgeführt.

Die Umsetzung der Verbindungen der allgemeinen Formel (XII) erfolgt mit Methylenchlorid unter Rückfluß.

Als aktivierende Reagenzien eignen sich vorzugsweise Trifluormethansulfonsäurechlorid, Mesylchlorid, Oxalylchlorid und Thionylchlorid. Besonders bevorzugt ist Thionylchlorid.

Die Umsetzung zu den Verbindungen der allgemeinen Formel (XIVa) erfolgt im ersten Schritt bevorzugt in Tetrahydrofuran und Triethylamin, im zweiten Schritt im System Wasser/Salzsäure. Die Reaktion wird in einem Temperaturbereich von 30°C bis 70°C durchgeführt.

Als Säuren für die Herstellung der erfindungsgemäßen Verbindungen der allgemeinen Formel (XV) wird besonders bevorzugt konzentrierte Schwefelsäure eingesetzt. Die Herstellung wird mit Methylenchlorid durchgeführt.

Im weiteren Aufarbeitungsgang wird als Base Kaliumcarbonat eingesetzt. Die Umsetzung erfolgt in einem Temperaturbereich von 0°C bis +20°C, besonders bevorzugt bei 10°C.

Die Halogenierung der Verbindungen der allgemeinen Formel (XV) erfolgt mit N-Bromsuccinimid in Methylenchlorid in Anwesenheit von Azobisisobutyronitril.

Im allgemeinen setzt man die Base in einer Menge von 0,05 mol bis 10 mol, bevorzugt von 1 mol bis 2 mol, jeweils bezogen auf 1 mol der Verbindungen der allgemeinen Formeln (IV), (VIII), (XI) und (XIVa) ein.

Die erfindungsgemäßen Verfahren werden im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel ( III) sind an sich bekannt.

Die Verbindungen der allgemeinen Formeln (IV), (VIII) und (VIIIa) sind bekannt oder können in Analogie zu

bekannten Methoden hergestellt werden.

Die Verbindungen der allgemeinen Formel (V) sind teilweise bekannt oder neu und können dann in Analogie zu publizierten Methoden hergestellt werden.

Die Verbindungen der allgemeinen Formel (VII) sind als Species neu und werden aus der entsprechenden Säure hergestellt.

Die enantiomerenreinen Verbindungen der allgemeinen Formel (IX) sind mit Ausnahme von X = CH-isopropyl neu und können wie oben beschrieben hergestellt werden.

Die Verbindungen der allgemeinen Formeln (X), (XI), (XIa) und (XII) sind neu und können wie oben beschrieben hergestellt werden.

Die Verbindungen der allgemeinen Formeln (XIV) und (XIVa) sind teilweise bekannt oder nach üblichen Methoden herstellbar.

Die enantiomerenreinen Verbindungen der allgemeinen Formel (XV)und (XVI) sind neu und können wie oben beschrieben hergestellt werden.

Die Verbindungen der allgemeinen Formel (VI) sind neu und können wie oben beschrieben hergestellt werden.

Die erfindungsgemäßen Verbindungen der allgemeinen Formel (I) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von koronaren Herzerkrankungen, Herzinsuffizienz, Störungen der Hirnleistung, ischämischen Gehirnerkrankungen, Apoplex, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteine (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Prävention und Behandlung von Atherosklerose, der Fettsucht, Pankreatitis und der Obstipation eingesetzt werden.

**1. Hemmung der Freisetzung ApoB-100-assoziierter Lipoproteine**

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukaryontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorption an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von $H_2O_2$ in ein gefärbtes Produkt um. Nach Ansäuerung des Reaktionsgemisches mit $H_2SO_4$ wird die spezifische Lichtabsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtabsorption im Vergleich zur Kontrolle (Lösemittelkontrolle ohne Substanz) um 50% inhibiert ist.

| Bsp.-Nr. | Apo B $IC_{50}$[nM] |
|---|---|
| 2 | 1.3 |
| 18 | 1.9 |
| 24 | 0.6 |
| 36 | 1.1 |
| 54 | 0.7 |
| 57 | 2.7 |

**2. Bestimmung der VLDL-Sekretion in vivo am Hamster**

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i.p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer Wellenlänge von 492 nm in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

**3. Hemmung der intestinalen Triglyceridabsoprtion in vivo (Ratten)**

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wäßrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus' entnommen. Anschließend werden die Traganth-Suspension, die Traganth-Olivenöl-Suspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung der Triglyceride erfolgt vollenzymatisch mit einem handelsüblichen UV-Test.

Der postprandiale Serumtriglyceridanstieg wird durch Subtraktion des Triglyceridvorwertes jeden Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs ($\Delta$TG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in $\Delta$% von der ölbelasteten Kontrolle angege-

ben.

$$\Delta\%\ \text{Triglyceridanstieg} = \frac{\Delta TG_{Substanz} - \Delta TG_{Traganthkontrolle}}{\Delta TG_{Ölbelastung} - \Delta TG_{Traganthkontrolle}} \times 100$$

Effekt von 1, 3 oder 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

| | **Serumtriglyceridanstieg in % (2 h pp)** |
|---|---|
| **Triglyceridbelastung** | 100 |
| **Traganthkontrolle** | 0 |

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant ($p < 0,05$) um mindestens 30 % vermindern, werden als pharmakologisch wirksam angesehen.

**4. Hemmung der VLDL-Sekretion in vivo (Ratte)**

Die Wirkung der Testsubstanzen auf die VLDL-Sekretion wird ebenfalls an der Ratte untersucht. Dazu wird Ratten 500 mg/kg Körpergewicht (2,5 mg/kg) Triton WR-1339, gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholesterinspiegels. Diese Anstiege können als Maß für die VLDL-Sekretionsrate herangezogen werden.

Den Tieren wird vor sowie zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die Erfindung betrifft außerdem die Kombination von neuen Pyrimido[1,2-b]indolen der allgemeinen Formel (I) mit einem Glucosidase- und/oder Amylasehemmer zur Behandlung von familiärer Hyperlipaemien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistat, AI-3688, Trestatin, Pradimicin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

**Abkürzungen:**

| | |
|---|---|
| Ac | = Acetyl |
| AIBN | = Azobisisobutyronitril |
| Bn | = Benzyl |
| Bz | = Benzoyl |
| cDec | = cycloDecyl |
| CDI | = N'-(3-Dimethylaminopropyl)-N-ethylcarbodiimid Hydrochlorid |
| cDodec | = cycloDodecyl |
| cHept | = cycloHeptyl |
| cHex | = cycloHexyl |
| cNon | = cycloNonyl |
| cOct | = cycloOctyl |
| cPent | = cycloPentyl |
| cPr | = cycloPropyl |
| 18-Crown-6 | = 1,4,7,10,13.16-Hexaoxacyclooctadecan |
| cUndec | = cycloUndecyl |
| DCC | = Dicyclohexylcarbodiimid |
| DDQ | = 2,3-Dichlor-5,6-dicyano-1,4-benzochinon |
| dia | = Diastereomer |
| dia A | = Diastereomer mit dem größeren $R_f$-Wert |
| dia B | = Diastereomer mit dem kleineren $R_f$-Wert |
| DMAP | = 4-(N,N-Dimethylamino)pyridin |
| DME | = 1,2-Dimethoxyethan |
| DMF | = N,N-Dimethylformamid |
| DMSO | = Dimethylsulfoxid |
| ent | = Enantiomer |
| Et | = Ethyl |
| HOBT | = 1-Hydroxy-1H-benzotriazol |
| iBu | = isoButyl |
| iPent | = isoPentyl |
| iPr | = isoPropyl |
| Me | = Methyl |
| Ment | = (L)-Menthyl |
| Mes | = Mesyl |
| NBS | = N-Bromsuccinimid |
| nBu | = normalButyl |
| nHex | = normalHexyl |
| nPent | = normalPentyl |
| nPr | = normalPropyl |
| Ph | = Phenyl |
| PPA | = Polyphosphorsäure |
| pTol | = paraTolyl |
| pTos | = paraTosyl |
| rac | = Racemat |
| sBu | = sekundärButyl |
| tBu | = tertiärButyl |

TFA = Trifluoressigsäure
THF = Tetrahydrofuran
TMS = Tetramethylsilan

Herstellungsvorschrift für das Laufmittel BABA:

87,9 ml einer wäßrigen 0,06667 molaren Kaliumdihydrogenphosphatlösung und 12,1 ml einer wäßrigen 0,06667 molaren Dinatriumhydrogenphosphatlösung werden gemischt. 60 ml der so zubereiteten Lösung werden mit 200 ml n-Butylacetat, 36 ml n-Butanol und 100 ml Eisessig geschüttelt, und die wäßrige Phase abgetrennt. Die organische Phase ist das Laufmittel BABA.

| Die verwendeten Lösemittelgemische: | |
|---|---|
| **Solvens** | **Bezeichnung** |
| Petrolether : Essigsäureethylester = 20:1 | A |
| Petrolether : Essigsäureethylester = 2:1 | B |
| Petrolether : Essigsäureethylester = 5:1 | C |
| Dichlormethan: Ethanol = 20:1 | D |
| Petrolether : Essigsäureethylester = 1:1 | E |
| Dichlormethan : Ethanol = 50:1 | F |
| Dichlormethan | G |
| Petrolether : Essigsäureethylester = 9:1 | H |
| Dichlormethan : Methanol = 19:1 | I |
| Petrolether : Essigsäureethylester = 4:1 | J |
| Dichlormethan : Methanol = 100:1 | K |
| Dichlormethan : Methanol = 100:3 | L |
| Toluol | M |
| Toluol : Essigester = 9:1 | N |
| Toluol : Essigsäureethylester = 2:1 | O |
| Petrolether : Essigester = 10:1 | P |
| Petrolether : Essigester = 20:1 | Q |
| Petrolether | R |
| Petrolether : Essigsäureethylester = 6:1 | XA |

**Ausgangsverbindungen**

**Beispiel I**

4-Tolyl-essigsäure-methylester

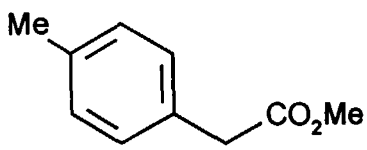

300 g (1,998 mol) 4-Tolyl-essigsäure werden in 2,5 l Methanol gelöst, mit 100 ml konz. Schwefelsäure verrührt und

2,5 h unter Rückfluß gekocht. Darauf rührt man nach und nach insgesamt 430 g (5,1 mol) Natriumhydrogencarbonat ein (Kohlendioxidentwicklung !), dampft das Methanol im Vakuum weitgehend ab, verteilt zwischen Wasser und Dichlormethan und extrahiert die wäßrige Phase mit Dichlormethan nach. Die vereinigten organischen Phasen werden mit Natriumsulfat getrocknet und im Vakuum vom Lösemittel befreit. Der Rückstand wird im Hochvakuum destilliert.

Ausbeute: 336 g
Siedetemperatur: 65°C (0,5 mbar)
$R_f$ = 0,81 (Toluol : Essigsäureethylester = 2:1) = 0

**Beispiel II**

4-Tolyl-essigsäureethylester

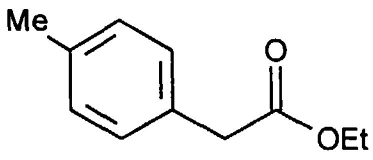

Ausgehend von 4-Tolyl-essigsäure wird der 4-Tolyl-essigsäureethylester analog der Vorschrift aus Beispiel I hergestellt.

$R_f$ = 0,43 (A)

**Beispiel III**

4-Methylphenylessigsäure-tert.butylester

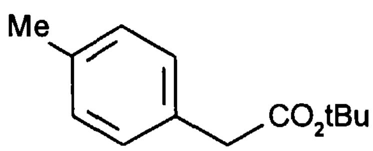

450 g (3 mol) 4-Methylphenylessigsäure, 1,13 l (12 mol) tert.Butanol und 90 g (0,74 mol) Dimethylaminopyridin (DMAP) werden in 2 l Dichlormethan gelöst.

Nach Zugabe von 680 g (3,3 mol) Dicyclohexylcarbodiimid (DCC), gelöst in 400 ml Dichlormethan, wird 20 h bei 25°C gerührt. Der ausgefallene Harnstoff abgesaugt, mit 200 ml Dichlormethan gewaschen und die organische Phase je zweimal mit 500 ml 2 M Salzsäure und Wasser gewaschen. Die organische Phase wird mit Natriumsulfat getrocknet, eingeengt und destilliert.

Ausbeute: 408 g (66%)
Siedepunkt: 73-78°C (0,2 Torr)

**Beispiel IV**

(2R/2S)-2-Cyclopentyl-2-(4-methylphenyl)essigsäure-tert.butylester

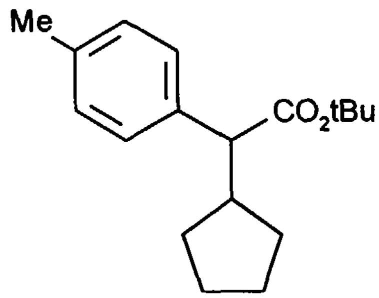

33,5 g (0,3 mol) Kalium-tert.butylat werden in 100 ml DMF unter Feuchtigkeitsausschluß bei 0°C vorgelegt, und 51,6 g (0,25 mol) der Verbindung aus Beispiel III in 250 ml DMF zugetropft. Es wird 30 min bei 0°C gerührt, 32,2 ml (0,3 mol) Cyclopentylbromid in 150 ml DMF bei 5-15°C zugetropft und 20 h bei 25°C gerührt. Nach Einengen wird der Rückstand zwischen Wasser/Diethylether verteilt, die Etherphase über Natriumsulfat getrocknet und eingeengt. Das Produkt kristallisiert aus.

Ausbeute: 67 g (97,5%)
Festpunkt: 51-53°C

Die racemischen Verbindungen der Tabelle I werden analog der Vorschrift von Beispiel IV hergestellt:

**Tabelle I:**

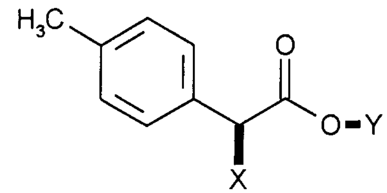

| Bsp.-Nr. | X | Y | a) Fp. (°C) b) $R_f$ (Solvens) | Spektren | Ausgangsmaterial aus Bsp.-Nr. |
|---|---|---|---|---|---|
| V | $CH_3$ = Me | $C(CH_3)_3$ = tBu | b) 0,71 (M) | | III |
| VI | $C_2H_5$ = Et | tBu | b) 0,67 (M) | | III |
| VII | $CH_2CH_2CH_3$ = nPr | tBu | b) 0,69 (M) | | III |
| VIII | $CH(CH_3)_2$ = iPr | Me | b) 0,86 (O) | | I |
| IX | $CH(CH_3)_2$ = iPr | tBu | b) 0,76 (N) | | III |
| X | $CH_2CH_2CH_2CH_3$ = nBu | tBu | b) 0,74 (M) | | III |
| XI | $CH_2CH(CH_3)_2$ = iBu | tBu | b) 0,70 (M) | | III |
| XII | $CH_2CH_2CH_2CH_2CH_3$ = nPent | tBu | b) 0,75 (H) | | III |
| XIII | $CH_2CH_2$-$CH(CH_3)_2$ = iPent | tBu | b) 0,54 (P) | | III |
| XIV | $CH(CH_2CH_3)_2$ | tBu | | MS: 276 ($M^+$, 4%) | III |
| XV | $CH_2CH_2CH_2CH_2CH_2CH_3$ = nHex | tBu | b) 0,75 (M) | | III |
| XVI | $CH_2CH(CH_2CH_3)_2$ | tBu | | MS: 290 ($M^+$, 1%) | III |

**Tabelle I - Fortsetzung**

| Bsp.-Nr. | —X | —Y | a) Fp. (°C)<br>b) $R_f$ (Solvens) | Spektren | Ausgangsmaterial aus Bsp.-Nr. |
|---|---|---|---|---|---|
| XVII | 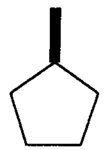 = cPent | Me | b) 0,59 (P) | | I |
| XVIII | 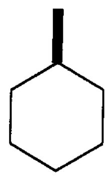 = cHex | Me | b) 0,62 (Q) | | I |
| XIX | cHex | tBu | b) 0,72 (M) | | III |
| XX | 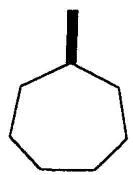 = cHept | Me | b) 0,57 (M) | | I |
| XXI | cHept | tBu | b) 0,67 (M) | | III |
| XXII | 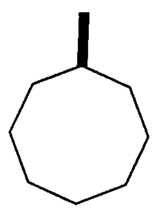 = cOct | tBu | b) 0,77 (M) | | III |

**Tabelle I - Fortsetzung**

| Bsp.-Nr. | ▬X | ▬Y | a) Fp. (°C) b) $R_f$ (Solvens) | Spektren | Ausgangsmaterial aus Bsp.-Nr. |
|---|---|---|---|---|---|
| XXIII | 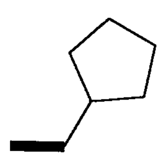 | tBu | b) 0,86 (Q) | | III |
| XXIV | 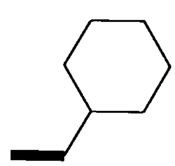 | tBu | b) 0,82 (N) | | III |

Die Verbindungen der Tabelle II werden analog der Vorschrift von Beispiel IV hergestellt; es werden lediglich 2,5 Äquivalente der Base und 2,5 Äquivalente des Halogenalkans (im Falle der cyclischen Alkylreste 1,2 Äquivalente des $\alpha,\omega$-Dihalogenalkans) eingesetzt.

**Tabelle II**

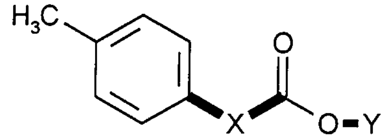

| Bsp.-Nr. | 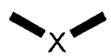 | 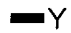 | a) Fp. (°C)<br>b) $R_f$ (Solvens) | Spektren | Ausgangsmaterial aus Bsp.-Nr. |
|---|---|---|---|---|---|
| XXV | 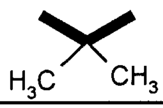 | tBu | b) 0,68 (F) | | III |
| XXVI | 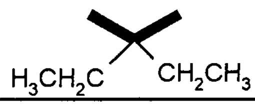 | tBu | b) 0,32 (R) | | III |
| XXVII | 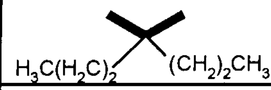 | tBu | b) 0,84 (B) | | III |
| XXVIII | 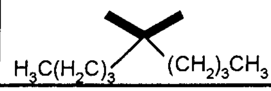 | tBu | b) 0,82 (C) | | III |
| XXIX | 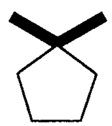 | tBu | b) 0,23 (R) | | III |

**Tabelle II - Fortsetzung**

| Bsp.-Nr. | 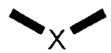 | 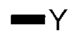 | a) Fp. (°C)<br>b) $R_f$ (Solvens) | Spektren | Ausgangsmaterial aus Bsp.-Nr. |
|---|---|---|---|---|---|
| XXX | 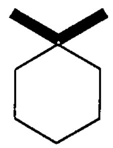 | tBu | b) 0,21 (R) | | III |
| XXXI | 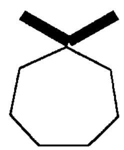 | tBu | b) 0,26 (R) | | III |

**Beispiel XXXII**

(2R/2S)-2-(4-Brommethyl-phenyl)-2-cyclopentyl-essigsäure-tert.butylester

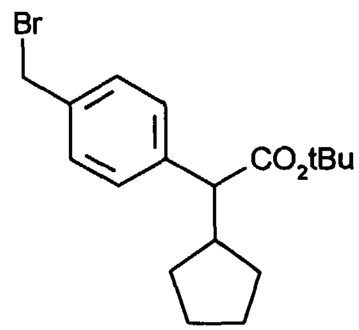

27,4 g (0,1 mol) der Verbindung aus Beispiel IV werden in 200 ml Tetrachlorkohlenstoff gelöst und zum Sieden erhitzt. Nach Zugabe von 0,82 g Azobisisobutyronitril (AIBN) werden 18,7 g (0,105 mol) N-Bromsuccinimid (NBS) portionsweise (NBS) zugegeben und anschließend 1 h refluxiert, auf 0°C abgekühlt und vom Succinimid abfiltriert. Nach Einengen des Filtrates fällt das Produkt aus. Es wird mit Petrolether (40/60) gewaschen und getrocknet.

Ausbeute: 20 g (57%)
Festpunkt: 73-76°C

Die racemischen Verbindungen der Tabelle III werden analog der Vorschrift von Beispiel-Nr. XXXII hergestellt:

Tabelle III:

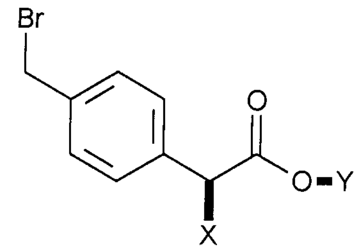

| Bsp.-Nr. | ▬X | ▬Y | a) Fp. (°C)<br>b) $R_f$ (Solvens) | Spektren | Ausgangsmaterial aus Bsp.-Nr. |
|---|---|---|---|---|---|
| XXXIII | ▬H | Me | b) 0,45 (XA) | | I |
| XXXIV | ▬H | tBu | b) 0,54 (H) | MS: 302, 304 [M + $NH_4$]$^+$; 100%, 98%) | III |
| XXXV | Me | tBu | b) 0,78 (M) | | V |
| XXXVI | Et | tBu | b) 0,75 (M) | | VI |
| XXXVII | nPr | tBu | b) 0,80 (M) | | VII |
| XXXVIII | iPr | Me | b) 0,78 (G) | | VIII |
| XXXIX | iPr | tBu | b) 0,90 (N) | | IX |
| XL | nBu | tBu | b) 0,82 (M) | | X |
| XLI | iBu | tBu | b) 0,86 (G) | | XI |
| XLII | nPent | tBu | b) 0,73 (H) | | XII |
| XLIII | iPent | tBu | | MS: 372, 374 [M+$NH_4$]$^+$; 79%, 77%) | XIII |
| XLIV | ▬$CH(CH_2CH_3)_2$ | tBu | | MS: 372, 372 ([M+$NH_4$]$^+$; 4%, 4%) | XIV |
| XLV | nHex | tBu | b) 0,85 (M) | | XV |

Tabelle III - Fortsetzung

| Bsp.-Nr. | —X | —Y | a) Fp. (°C)<br>b) $R_f$ (Solvens) | Spektren | Ausgangsmaterial aus Bsp.-Nr. |
|---|---|---|---|---|---|
| XLVI | $-CH_2CH(CH_2CH_3)_2$ | tBu | | MS: 386, 388 ($[M+NH_4]^+$; 14%, 14%) | XVI |
| XLVII | cPent | Me | b) 0,63 (P) | | XVII |
| XLVIII | cHex | Me | b) 0,47 (Q) | | XVIII |
| IL | cHex | tBu | b) 0,58 (P) | | XIX |
| L | cHept | Me | b) 0,59 (M) | | XX |
| LI | cHept | tBu | b) 0,84 (G) | | XXI |
| LII | cOct | tBu | b) 0,49 (Q) | | XXII |
| LIII | 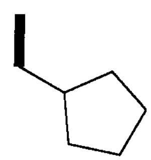 | tBu | b) 0,58 (A) | | XXIII |
| LIV | 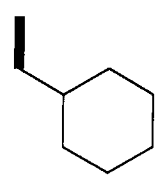 | tBu | | $^1H$-NMR (250 MHz, $CDCl_3$, TMS): $\delta$ = 3,58 (M,1H), 4,49 (S,2H) ppm | XXIV |

**Tabelle III - Fortsetzung**

Die Verbindungen der Tabelle IV werden analog der Vorschrift von Beispiel XXXII hergestellt:

**Tabelle IV**

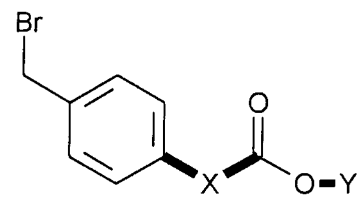

| Bsp.-Nr. | 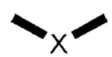 | —Y | a) Fp. (°C) b) $R_f$ (Solvens) | Spektren | Ausgangsmaterial aus Bsp.-Nr. |
|---|---|---|---|---|---|
| LV | 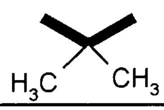 | tBu | b) 0,68 (F) | | XXV |
| LVI | 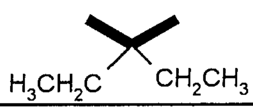 | tBu | b) 0,38 (Q) | | XXVI |
| LVII | 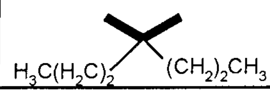 | tBu | b) 0,84 (B) | | XXVII |
| LVIII | 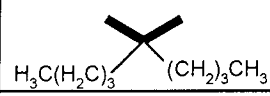 | tBu | b) 0,82 (C) | | XXVIII |

Tabelle IV - Fortsetzung

| Bsp.-Nr. | 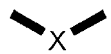 | 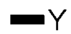 | a) Fp. (°C)<br>b) $R_f$ (Solvens) | Spektren | Ausgangsmaterial aus Bsp.-Nr. |
|---|---|---|---|---|---|
| LIX | 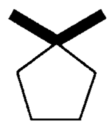 | tBu | | MS: 356, 358 ($[M+NH_4]^+$; 9%, 11%) | XXIX |
| LX | 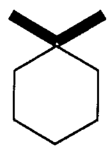 | tBu | | MS: 370, 372 ($[M+NH_4]^+$; 5%, 5%) | XXX |
| LXI | 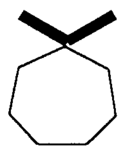 | tBu | b) 0,47 (Q) | | XXXI |

**Beispiel LXII**

(2R/2S)-2-(2-Nitrophenyl)-2-cyano-essigsäureethylester

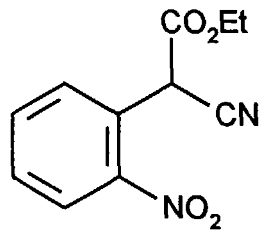

93,4 g (832 mmol) Kalium-tert.butanolat und 92,4 ml (868 mmol) Cyanessigsäureethylester werden mit 680 ml tert.Butanol bei ca. 20°C verrührt [vgl. C.A. Grob und O. Weissbach, Helv. Chim. Acta 44, 1748 (1961)]. Nach 30 Minuten läßt man eine auf ca. 60°C vorgewärmte Lösung von 63 g (400 mmol) 2-Chlornitrobenzol in 150 ml tert.Butanol zufließen und kocht 10 Stunden unter Rückfluß. Die auf ca. 20°C abgekühlte Reaktionslösung wird mit 2 M Salzsäure auf einen pH-Wert von 3 gestellt und anschließend im Wasserbad von 40°C eingedampft (30 mbar). Der Rückstand wird auf 1 l Diethylether und 500 ml Wasser gegossen und die abgetrennte organische Phase mehrfach mit wäßriger Natriumhydrogencarbonatlösung und anschließend mit Wasser nachgewaschen. Die mit Magnesiumsulfat getrocknete Lösung wird eingedampft und das rohe Produkt an Kieselgel 60 (Merck / 40-63 µm / Petrolether : Essigsäureethylester = 3:1) chromatographisch aufgereinigt.

Ausbeute: 67,7 g (289 mmol) 72% d.Th.
DC: $R_f$ = 0,46 (B)
$^1$H-NMR ($CDCl_3$, 250 MHz, TMS): δ = 1,33 (t, 3H); 4,31 (q, 2H); 5,68 (s, 1H); 7,65 (m, 1H); 7,73 - 7,81 (m, 2H); 8,23 (m, 1H) ppm.

Die racemischen Verbindungen der Tabelle V werden analog der Vorschrift des Beispiels LXII hergestellt

**Tabelle V:**

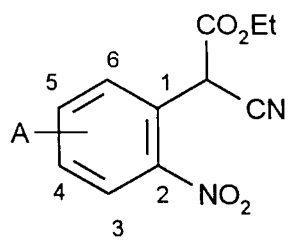

| Bsp.-Nr. | A | a) Fp. (°C)<br>b) $R_f$ (Solvens) | Spektren | Ausgangs-material aus Bsp.-Nr. |
|---|---|---|---|---|
| LXIII | 4-Cl | | MS (CI, $NH_3$):<br>286 ($[M+NH_4]^+$; 73%) | |
| LXIV | 5-OMe | b) 0,53 (G) | | |
| LXV | 4-OMe | b) 0,47 (G) | | |

**Beispiel LXVI**

2-Amino-3-ethoxycarbonyl-indol

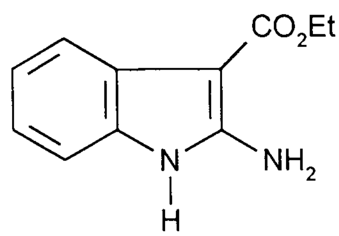

20 g (85,4 mmol) der Verbindung aus Beispiel LXII werden in 300 ml Essigsäure auf 100°C erhitzt, portionsweise unter starkem Rühren mit insgesamt 15 g (268 mmol) Eisenpulver versetzt und 45 Minuten unter Rückfluß gekocht [vgl. C.A. Grob und O. Weissbach, Helv. Chim. Acta 44, 1748 (1961)]. Nach dem Abkühlen auf ca. 20°C wird das Gemisch über einen Seitz-Filter abgesaugt und mit 100 ml Essigsäure nachgewaschen. Das Filtrat wird weitgehend eingedampft, mit Diethylether und Wasser wieder aufgenommen und die organische Phase mit wäßriger Kaliumhydrogencarbonatlösung extrahiert. Die gesammelten wäßrigen Phasen werden mit 2 M Salzsäure auf pH = 3-4 gestellt und mit Diethylether ausgeschüttelt. Schließlich werden die vereinigten organischen Phasen mit Magnesiumsulfat getrocknet und eingedampft. Das Rohprodukt wird chromatographisch an Kieselgel 60 (Merck / 40-63 µm / Petrolether : Essigsäureethylester = 2:1 bis 1:1) gereinigt.

Ausbeute: 9,9 g (48,5 mmol) 57% d.Th.
DC: $R_f$ = 0,35 (E)
$^1$H-NMR ($d_6$-DMSO, 200 MHz, TMS): δ = 1,32 (t, 3H); 4,21 (q, 2H); 6,68 (s, br, 2H); 6,82 - 6,92 (m, 2H); 7,10 (m, 1H); 7,54 (m, 1H); 10,64 (s, br, 1H) ppm.

**Beispiel LXVII**

2-Amino-indol Hydrochlorid

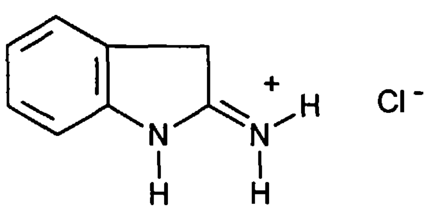

30 g (147 mmol) der Verbindung aus Beispiel LXVI werden 2 Stunden bei 100°C in 350 ml konzentrierter wäßriger Salzsäure gerührt (Kohlendioxidentwicklung) [vgl. R.A. Glennon undM. von Strandtmann, J. Heterocycl. Chem. 12, 135 (1975)]. Das Reaktionsgemisch wird zur Trockene eingedampft und mit einem Lösemittelgemisch von Ethanol : Diethylether = 1:1 ausgerührt. Der 1. Niederschlag wird abgesaugt, das Filtrat wie zuvor mit einer nun kleineren Lösemittelmenge ausgerührt und wieder abgesaugt (2. Niederschlag). Die Produktchargen werden im Hochvakuum über Phosphorpentoxid getrocknet. Ausbeute:

1. Nd.: 19,96g (118 mmol) 81% d.Th.
2. Nd.: 2,28 g ( 14 mmol) 9% d.Th.
DC: $R_f$ = 0,33 (BABA)
$^1$H-NMR ($d_6$-DMSO, 300 MHz, TMS): δ = 4,19 (s, 2H); 7,13 (m, 1H); 7,2 (m, 1H); 7,31 (m, 1H); 7,42 (m, 1H); 9,95 (s, br, 1H); 10,17 (s, br, 1H); 12,39 (s, br, 1H) ppm.

**Beispiel LXVIII**

2-Nitro-5-chloro-benzylcyanid

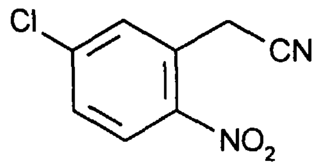

300 ml flüssiges Ammoniak werden vorgelegt und unter Rühren mit 20 g (0,13 mol) p-Chlornitrobenzol in 125 ml Ether und 25 g (0,65 mol) gepulvertem Natriumhydroxid versetzt. Nach 10 min. Rühren bei ca. -35°C wird innerhalb von 40 min bei dieser Temperatur 10,5 g (0,14 mol) Chloracetonitril in 25 ml Ether zu dieser Suspension getropft. Nach 2,5 h wird die Reaktion durch die Zugabe von 25 g festem Ammoniumchlorid beendet, der Ammoniak abgedampft und das Volumen durch Zugabe von Tetrachlormethan in etwa konstant gehalten. Anschließend wird der Ether weitgehend abdestilliert und die Reaktionsmischung warm filtriert. Der Rückstand wird zweimal mit je 300 ml heißem Tetrachlormethan extrahiert, die vereinigten Filtrate eingeengt. Dieser Rückstand wird dreimal mit je 125 ml heißem Cyclohexan extrahiert und der Rückstand chromatographisch an Kieselgel gereinigt.

Ausbeute: 5 g (20% , Lit. 75%)
$R_f$ = 0,32 (Petrolether: Essigsäureethylester 10:1) = P
$^1$H-NMR (200 MHz, $CDCl_3$): δ = 4,22 (s, 2H); 7,56 (dd, 1H); 7,76 (d, 1H); 8,19 (d, 1H) ppm.
MS (Cl, $NH_3$): 214 ($[M+NH_4]^+$, 100%)

**Beispiel LXIX**

2-Nitro-5-methoxybenzylcyanid

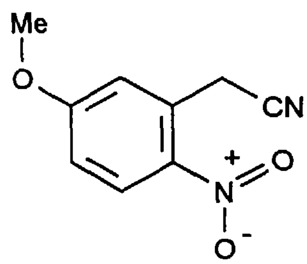

44,0 g (0,167 mol) der Verbindung aus Beispiel LXIV werden in 450 ml 1 N Natriumcarbonat-Lösung 18 h bei 50°C gerührt. Nach dem Abkühlen auf Raumtemperatur wird der Niederschlag abgesaugt und im Vakuum (0,1 Torr) bei 50°C getrocknet.

Ausbeute: 21,6 g (67%)
Fp.: 78°C
$^1$H-NMR (200 MHz, $CDCl_3$): δ = 3,95 (s, 3H); 4,26 (s, 2H); 6,98 (dd, 1H); 7,18 (d, 1H), 8,27 (d, 1H) ppm.
MS (Cl, $NH_3$): 210 ($[M+NH_4]^+$, 100%)

Die Verbindungen der Tabelle VI werden analog der Vorschrift von Beispiel LXIX hergestellt:

## Tabelle VI

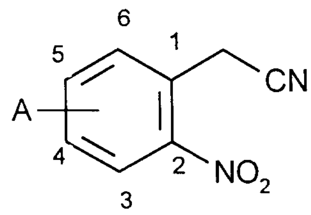

| Bsp.-Nr. | A | a) Fp. (°C)<br>b) $R_f$ (Solvens) | Spektren | Ausgangsmaterial aus Bsp.-Nr. |
|---|---|---|---|---|
| LXX | 4-Cl | | MS (CI, $NH_3$): 214 ($[M+NH_4]^+$, 100%) | LXIII |
| LXXI | 4-OMe | a) 62°C | | LXV |

**Beispiel LXXII**

2-Amino-5-chloro-benzylcyanid

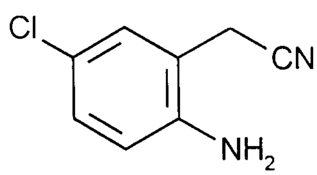

5,0 g (0,025 mol) der Verbindung aus Beispiel LXVIII und 4,6 g (0,039 mol) Zinn werden in 40ml Isopropanol suspendiert und unter Argon bei 10°C langsam mit 21 ml konz. Salzsäure versetzt. Nach vollständiger Zugabe wird das Kältebad entfernt, so daß die Innentemperatur 35-40°C erreicht. Nach etwa 1,5 h ist das Zinn nahezu aufgelöst und Zusatz von 1 N Natronlauge zu einer Probe der Reaktionslösung verursacht keine Blaufärbung mehr. Das Reaktionsgemisch wird dann auf etwa 15 ml eingeengt, filtriert, der Rückstand in 50 ml Wasser gelöst und erneut filtriert. Das Filtrat wird bei 5°C mit ca. 12 ml 30%iger Natronlauge auf pH 8,0 gestellt, der Niederschlag abfiltriert und zweimal mit je 25 ml Wasser gewaschen. Der Nutschkuchen wird dann dreimal mit je 25 ml kochendem Isopropanol extrahiert und das Filtrat im Vakuum eingeengt und getrocknet.

Ausbeute: 2,5 g (60%)
$^1$H-NMR (200 MHz, $CDCl_3$): δ = 3,55 (s, 2H); 3,70 (s, breit, 2H); 6,69 (d, 1H); 7,14 (dd, 1H); 7,21 (d, 1H) ppm.
MS (EI): 166 ($M^+$, 74%)

Die Verbindungen der Tabelle VII werden analog der Vorschrift von Beispiel LXXII hergestellt:

## Tabelle VII

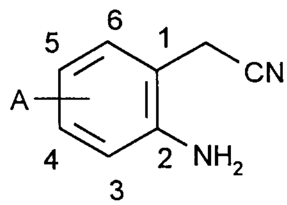

| Bsp.-Nr. | A | a) Fp. (°C) b) $R_f$ (Solvens) | Spektren | Ausgangsmaterial aus Bsp.-Nr. |
|---|---|---|---|---|
| LXXIII | 4-Cl | a) 85°C | | LXX |
| LXXIV | 5-OMe | | MS (CI, $NH_3$): 163 ($[M+H]^+$, 100%) | LXIX |
| LXXV | 4-OMe | | MS (CI, $NH_3$): 163 ($[M+H]^+$, 100%) | LXXI |

**Beispiel LXXVI**

2-Amino-5-chlor-indol Hydrochlorid

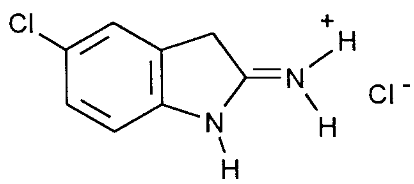

1,2 g (0,053 mol) Natrium werden in 30 ml Isopropanol gelöst. Zu dieser Lösung werden bei ca.80°C 2,5 g (0,015 mol) der Verbindung aus Beispiel Nr. LXXII in 15 ml Isopropanol zugegeben und anschließend für 1,5 h unter Rückfluß gekocht. Die Reaktionslösung wird bei 60°C mit 15 ml Wasser versetzt, das Isopropanol im Vakuum weitgehend entfernt und der Niederschlag abfiltriert. Dieser wird dann in 12 ml Isopropanol aufgenommen, mit 1 ml Wasser versetzt und mit ethanolischer

Salzsäure auf pH 2,0 gestellt. Nach erneutem Einengen wird aus Wasser - Aceton umkristallisiert und im Vakuum getrocknet.

Ausbeute: 1,6 g (53%)
$^1$H-NMR (200 MHz, $CDCl_3$): $\delta$ = 4,22 (s, 2H); 7,24 (d, 1H); 7,37 (d, 1H); 7,51 (d, 1H); 10,18 (s, 1H); 10,40 (s, 1H); 12,58 (s, 1H) ppm.
MS (EI): 166 ($M^+$, 100%).

Die Verbindungen der Tabelle VIII werden analog der Vorschrift von Beispiel-Nr. LXXVI hergestellt:

**Tabelle VIII**

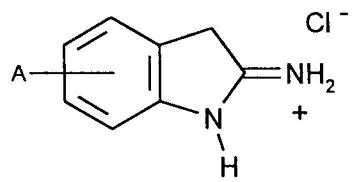

| Bsp.-Nr. | A | a) Fp. (°C) b) $R_f$ (Solvens) | Spektren | Ausgangs-material aus Bsp.-Nr. |
|---|---|---|---|---|
| LXXVII | 6-Cl | a) >200°C | MS (CI, $NH_3$): 167 ([M+H]$^+$, 100%) | LXXIII |
| LXXVIII | 5-OMe | a) >200°C | MS (CI, $NH_3$): 163 ([M+H]$^+$, 100%) | LXXIV |
| LXXIX | 6-OMe | a) >200°C | MS (CI, $NH_3$): 163 ([M+H]$^+$, 100%) | LXXV |

**Beispiel LXXX**

2,4-Dimethyl-pyrimido[1,2-d]indol

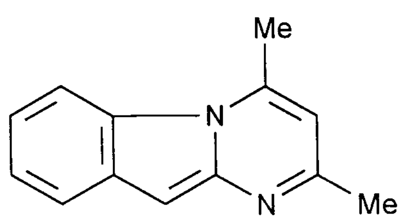

22 g (131 mmol) der Verbindung aus Beispiel LXVII werden bei ca. 20°C in 200 ml Pyridin mit 16,7 ml (196 mmol) 2,4-Pentandion umgesetzt [vgl. A.N. Kost, R.S. Sagitullin, V.I. Gorbunov und N.N. Modyanov, Khim. Geterosikl. Soedin 6, 359-363 (1970); engl. Übersetzung 334-337]. Nach 20 Stunden wird das Reaktionsgemisch auf 1,2 l Wasser gegossen und der anfallende Niederschlag abgesaugt. Der rohe Feststoff wird mehrfach mit Wasser gewaschen, scharf abgesaugt und im Hochvakuuum über Phosphorpentoxid getrocknet.

Ausbeute: 22,5 g (115 mmol) 88% d.Th.
DC: $R_f$ = 0,31 (B)
$^1$H-NMR ($CDCl_3$, 300 MHz, TMS): δ = 2,51 (s, 3H); 2,93 (s, 3H); 6,19 (s, 1H); 6,77 (s, 1H); 7,25 (m, 1H); 7,38 (m, 1H); 7,83 (m, 1H); 8,05 (m, 1H) ppm.

Die Verbindungen der Tabelle IX werden analog der Vorschrift von Beispiel LXXX hergestellt:

Tabelle IX

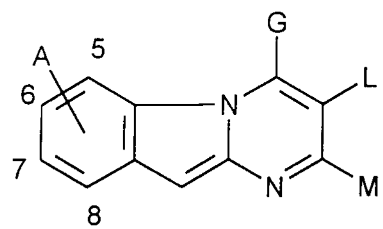

| Bsp.-Nr. | A | G | L | M | a) Fp. (°C) b) $R_f$ (Solvens) | Spektren | Ausgangs-material aus Bsp.-Nr. |
|---|---|---|---|---|---|---|---|
| LXXXI | H | Me | H | Et | b) 0,39 (L) | | LXVII |
| LXXXII | H | Et | H | Me | b) 0,31 (L) | | LXVII |
| LXXXIII | H | Et | H | Et | | MS (CI): 224 ($M^+$, 100%) | LXVII |
| LXXXIV | H | Me | Me | Me | b) 0,60 (B) | | LXVII |
| LXXXV | H | Me | Et | Me | b) 0,48 (B) | | LXVII |
| LXXXVI | H | Et | Me | Me | b) 0,75 (B) | | LXVII |
| LXXXVII | H | Me | Me | Et | b) 0,60 (B) | | LXVII |
| LXXXVIII | 6-Cl | Me | H | Me | a) 155°C | | LXXVI |
| LXXXIX | 7-Cl | Me | H | Me | | MS (CI, $NH_3$): 231 ($[M+H]^+$, 100%) | LXXVII |
| XC | 6-OMe | Me | H | Me | b) 0,85 (I) | | LXXVIII |
| XCI | 7-OMe | Me | H | Me | a) 76°C | | LXXIX |

**Beispiel XCII**

2(R/S)-Cyclopentyl-2-[4-(2,4-dimethyl-pyrimido[1,2-a]indol-9-yl-methyl)phenyl]essigsäure-tert.butylester

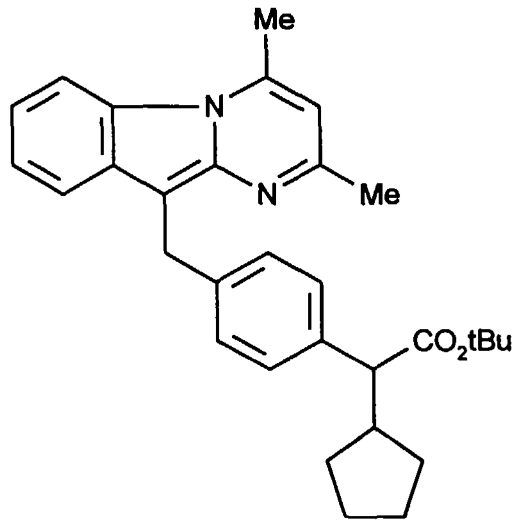

6,57 g (33,5 mmol) der Verbindung aus Beispiel LXXX, 26,1 g (50,2 mmol) der Verbindung aus Beispiel XXXII und 10,9 g (33,46 mmol) Cäsiumcarbonat werden in 70 ml wasseifreiem N,N-Dimethylformamid (DMF) unter Argon bei ca. 20°C zusammengegeben und 45 Minuten in einem Ölbad von 120°C gerührt. Das abgekühlte Reaktionsgemisch wird auf kaltes Wasser gegossen und mit Diethylether extrahiert. Die organische Phase wird mit Magnesiumsulfat getrocknet und eingedampft. Das so erhaltene Rohprodukt wird mit Methanol ausgerührt, abgesaugt, mit Methanol nachgewaschen und im Hochvakuum über Phosphorpentoxid getrocknet.

1. Ausbeute: 8.02 g (17,1 mmol) 51% d.Th. Das eingedampfte Filtrat wird chromatographisch an Kieselgel 60 (Merck / 40-63 µm / Petrolether : Essigsäureethylester = 10:1 bis 5:1) gereinigt.
2. Ausbeute: 1,40 g (3,0 mmol) 9% d.Th.
DC: $R_f$ = 0,44 (C)
$^1$H-NMR ($CDCl_3$, 200 MHz, TMS): δ = 0,93 (m, 1H); 1,36 (s, 9H); 1,14 - 1,71 (m, 6H); 1,85 (m, 1H); 2,38 (m, 1H); 2,51 (s, 3H); 2,92 (s, 3H); 3,06 (d, 1H); 4,46 (s, 2H); 6,15 (s, 1H); 7,12 - 7,34 (m, 6H); 7,63 (m, 1H), 8,05 (m, 1H) ppm.

Die racemischen Verbindungen der Tabelle X werden analog der Vorschrift von Beispiel XCII hergestellt:

Tabelle X

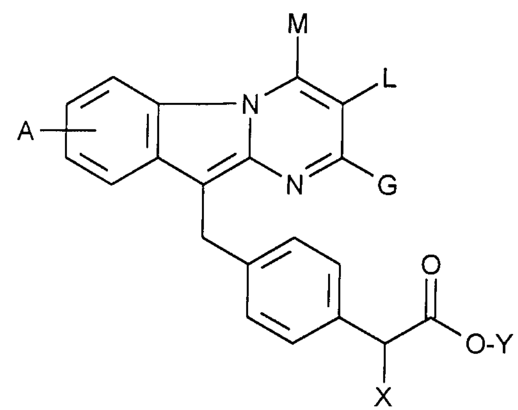

| Bsp.-Nr. | A | G | L | M | X | Y | a) Fp. (°C) b) $R_f$ (Solvens) | Spektren | Ausgangs-material aus Bsp.-Nr. |
|---|---|---|---|---|---|---|---|---|---|
| XCIII | H | Me | H | Me | H | tBu | b) 0,63 (J) | | LXXX und XXXIV |
| XCIV | H | Me | H | Me | Me | tBu | b) 0,28 (C) | | LXXX und XXXV |
| XCV | H | Me | H | Me | Et | tBu | b) 0,34 (C) | | LXXX und XXXVI |
| XCVI | H | Me | H | Me | nPr | tBu | b) 0,49 (C) | | LXXX und XXXVII |
| XCVII | H | Me | H | Me | nBu | tBu | b) 0,35 (C) | | LXXX und XL |
| XCVIII | H | Me | H | Me | iBu | tBu | b) 0,57 (B) | | LXXX und XLI |
| IC | H | Me | H | Me | nPent | tBu | | MS(CI, $NH_3$): 4J1 ($[M + H]^+$), 100%) | LXXX und XLII |

Tabelle X - Fortsetzung

| Bsp.-Nr. | A | G | L | M | X | Y | a) Fp. (°C) b) $R_f$ (Solvens) | Spektren | Ausgangsmaterial aus Bsp.-Nr. |
|---|---|---|---|---|---|---|---|---|---|
| C | H | Me | H | Me | iPent | tBu | b) 0,54 (J) | | LXXX und XLIII0 |
| CI | H | Me | H | Me | $-CH(Et)_2$ | tBu | b) 0,69 (J) | | LXXX und XLIV |
| CII | H | Me | H | Me | nHex | tBu | b) 0,36 (C) | | LXXX Und XLV |
| CIII | H | Me | H | Me | $-CH_2CH(Et)_2$ | tBu | b) 0,69 (J) | | LXXX und XLVI |
| CIV | H | Me | H | Me | cHex | tBu | b) 0,27 (C) | | LXXX und IL |
| CV | H | Me | H | Me | cHex | Me | b) 0,51 (B) | | LXXX und XLVIII |
| CVI | H | Me | H | Me | cHept | tBu | b) 0,18 (C) | | LXXX und LI |
| CVII | H | Me | H | Me | cOct | tBu | b) 0,25 (C) | | LXXX und LII |
| CVIII | H | Me | H | Me | $-CH_2$-cPent | tBu | b) 0,42 (C) | | LXXX und LIII |
| CIX | H | Me | H | Me | 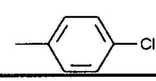 | Me | b) 0,43 (J) | | LXXX und |
| CX | H | Me | H | Et | cPent | tBu | b) 0,37 (H) | | LXXXI und XXXII |
| CXI | H | Et | H | Me | cPent | tBu | b) 0,82 (L) | | LXXXII und XXXII |
| CXII | H | Et | H | Et | cPent | tBu | b) 0,70 (B) | | LXXXIII und XXXII |
| CXIII | H | Me | Me | Me | cPent | tBu | b) 0,47 (K) | | LXXXIV und XXXII |
| CXIV | H | Me | Et | Me | cPent | Ment | b) 0,50 (C) | | LXXXV und CLXXII |
| CXV | H | Et | Me | Me | cPent | Ment | b) 0,54 (C) | | LXXXVI und CLXXII |

**Tabelle X - Fortsetzung**

| Bsp.-Nr. | A | G | L | M | X | Y | a) Fp. (°C)<br>b) $R_f$ (Solvens) | Spektren | Ausgangsmaterial aus Bsp.-Nr. |
|---|---|---|---|---|---|---|---|---|---|
| CXVI | H | Me | Me | Et | cPent | Ment | b) 0,50 (C) | | LXXXVI und CLXXII |
| CXVII | 6-Cl | Me | H | Me | cPent | tBu | a) 181°C | | LXXXVIII und XXXII |
| CXVIII | 7-Cl | Me | H | Me | cPent | tBu | | MS (CI, $NH_3$): 503 ($M^+$, 100%) | LXXXIX und XXXII |
| CXIX | 6-OMe | Me | H | Me | cPent | tBu | b) 0,87 (L) | | XC und XXXII |
| CXX | 7-OMe | Me | H | Me | cPent | tBu | b) 0,84 (L) | | XCI und XXXII |

**Beispiel CXXI**

2(R/S)-2-Cyclopentyl-2-[4-(2,4-dimethyl-pyrimido[1,2-a]indol-9-yl-methyl)phenyl]essigsäure

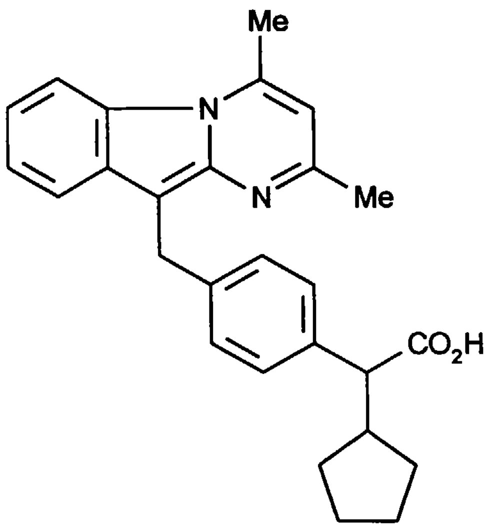

8,00 g (17,1 mmol) der Verbindung aus Beispiel XCII werden mit 14 ml konzentrierer Salzsäure in 60 ml Dioxan bei einer Badtemperatur von 70°C 1,5 h umgesetzt. Nach Abkühlen der Reaktionslösung wird mit 600 ml Wasser verdünnt und mit 2 M wäßriger Natronlauge auf einen pH-Wert von 2,5 gestellt. Der dabei anfallende Niederschlag wird abgesaugt, mehrfach mit Wasser gewaschen und im Hochvakuum über Phosphorpentoxid getrocknet.

Ausbeute: 6,89 g (16,7 mmol) 98% d.Th.
DC: $R_f$ = 0,41 (D)
$^1$H-NMR ($D_6$-DMSO, 200 MHz, TMS): δ = 0,89 (m, 1H); 1,08 - 1,61 (m, 6H); 1,78 (m, 1h); 2,37 (m, 1H); 2,46 (s, 3H); 2,96 (s, 3H); 4,32 (s, 2H); 6,46 (s, 1H); 7,11 - 7,37 (m,6H); 7,70 (m, 1H); 8,19 (m, 1H); 12,15 (s, br, 1H) ppm.

Die racemischen Verbindungen der Tabelle XI werden analog der Vorschrift von Beispiel CXXI hergestellt:

## Tabelle XI

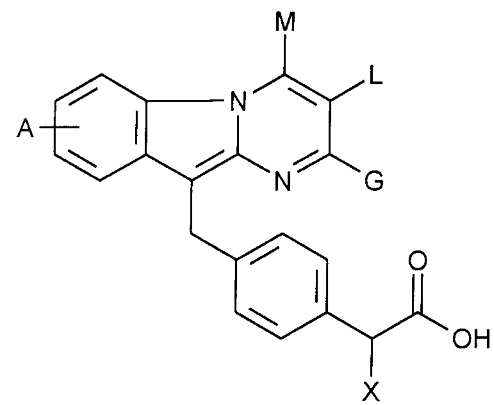

| Bsp.-Nr. | A | G | L | M | X | a) Fp. (°C)<br>b) $R_f$ (Solvens) | Spektren | Ausgangs-material aus Bsp.-Nr. |
|---|---|---|---|---|---|---|---|---|
| CXXII | H | Me | H | Me | H | | MS (CI, $NH_3$): 345 ($[M+H]^+$, 20%) | XCIII |
| CXXIII | H | Me | H | Me | Me | b) 0,23 (D) | | XCIV |
| CXXIV | H | Me | H | Me | Et | b) 0,38 (D) | | XCV |
| CXXV | H | Me | H | Me | nPr | b) 0,29 (D) | | XCVI |
| CXXVI | H | Me | H | Me | nBu | b) 0,17 (B) | | XCVII |
| CXXVII | H | Me | H | Me | iBu | b) 0,25 (B) | | XCVIII |
| CXXVIII | H | Me | H | Me | nPent | | MS (CI, $NH_3$: 415 ($[M+H]^+$, 100%) | IC |
| CXXIX | H | Me | H | Me | iPent | | MS (CI, $NH_3$): 414 ($M^+$, 100%) | C |
| CXXX | H | Me | H | Me | $CH(Et)_2$ | | MS (CI, $NH_3$): 415 ($[M+H]^+$, 100%) | CI |

## Tabelle XI - Fortsetzung

| Bsp.-Nr. | A | G | L | M | X | a) Fp. (°C) b) $R_f$ (Solvens) | Spektren | Ausgangs-material aus Bsp.-Nr. |
|---|---|---|---|---|---|---|---|---|
| CXXXI | H | Me | H | Me | nHex | b) 0,20 (B) | | CII |
| CXXXII | H | Me | H | Me | $-CH_2CH(Et)_2$ | | MS (CI, $NH_3$): 429 ($[M+H]^+$, 100%) | CIII |
| CXXXIII | H | Me | H | Me | cHex | b) 0,16 (B) | | CIV |
| CXXXIV | H | Me | H | Me | cHept | b) 0,52 (D) | | CVI |
| CXXXV | H | Me | H | Me | cOct | b) 0,45 (D) | | CVII |
| CXXXVI | H | Me | H | Me | $-CH_2$cPent | b) 0,22 (D) | | CVIII |
| CXXXVII | H | Me | H | Et | cPent | b) 0,13 (L) | | CX |
| CXXXVIII | H | Et | H | Me | cPent | b) 0,26 (L) | | CXI |
| CXXXIX | H | Et | H | Et | cPent | | MS (CI, $NH_3$) 441 ($[M+H\}^+$, 100% | CXII |
| CXL | H | Me | Me | Me | cPent | | MS (CI, $NH_3$) 427 ($[M+H\}^+$, 100% | CXIII |
| CXLI | 6-Cl | Me | H | Me | cPent | | MS (CI, $NH_3$) 447 ($[M+H\}^+$, 100% | CXVII |
| CXLII | 7-Cl | Me | H | Me | cPent | | MS (CI, $NH_3$) 447 ($[M+H\}^+$, 100% | CXVIII |
| CXLIII | 6-OMe | Me | H | Me | cPent | | MS (CI, $NH_3$) 443 ($[M+H\}^+$, 100% | CXIX |
| CXLIV | 7-OMe | Me | H | Me | cPent | | MS (CI, $NH_3$) 442 ($M^+$, 100% | CXX |

**Beispiel CXLV**

2-(S)-2-Cyclopentyl-2-[4-{(2,4-dimethyl-3-ethyl-pyrimido[1,2-a]indol-9-yl)methyl}phenyl]-essigsäure

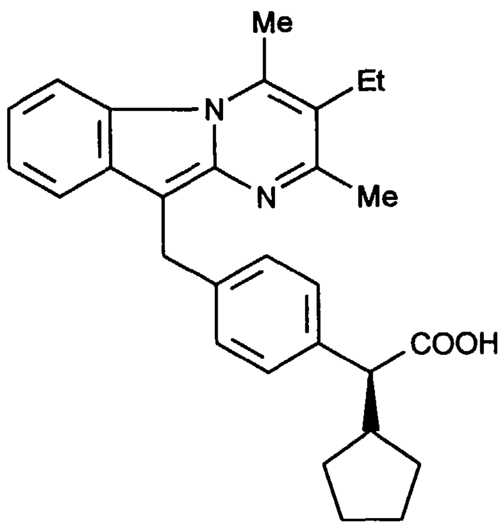

445 mg (0,769 mmol) der Verbindung aus Beispiel CXIV werden in 6 ml Ameisensäure und 3 ml 48%ige Bromwasserstoffsäure 4 h unter Rückfluß gekocht, danach auf Wasser gegossen und mit Natriumhydrogencarbonat auf pH = 2 gestellt. Der anfallende Niederschlag wird abgesaugt, mit Wasser gewaschen, scharf abgesaugt, mit Petrolether nachgewaschen und wieder scharf abgesaugt. Die Rohausbeute nach Vakuumtrocknung über Phosphorpentoxid beträgt 242 mg. Die Mutterlauge dieser Aufarbeitung wird mit Diethylether und Wasser aufgenommen, die wäßrige Phase mit Ether nachextrahiert, die vereinigten organischen Phasen mit wasserfreiem Magnesiumsulfat getrocknet und im Vakuum - zuletzt im Hochvakuum - vom Lösemittel befreit.

Rohausbeute: 50 mg
Die vereinigten Produktchargen weden chromatographisch an Kieselgel 60 (Merck / Dichlormethan : Ethanol = 100:1) gereinigt.
Ausbeute: 259 mg
$R_f$ = 0,30 (B)

Die Verbindungen der Tabelle XII werden analog der Vorschrift von Beispiel CXLV hergestellt:

Tabelle XII

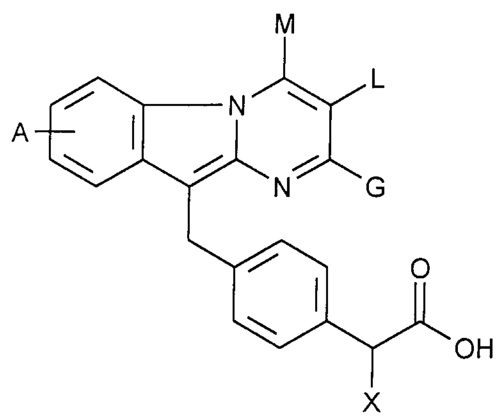

| Bsp.-Nr. | A | G | L | M | X | a) Fp. (°C)<br>b) $R_f$ (Solvens) | Spektren | Ausgangsmaterial aus Bsp.-Nr. |
|---|---|---|---|---|---|---|---|---|
| CXLVI | H | Et | Me | Me | cPent | b) 0,27 (B) | | CXV |
| CXLVII | H | Me | Me | Et | cPent | b) 0,18 (B) | | CXVI |

**Beispiel CXLVIII**

2(R/S)-2-Cyclohexyl-2-[4-(2,4-dimethyl-pyrimido[1,2-a]indol-9-yl)methyl)-phenyl]essigsäure

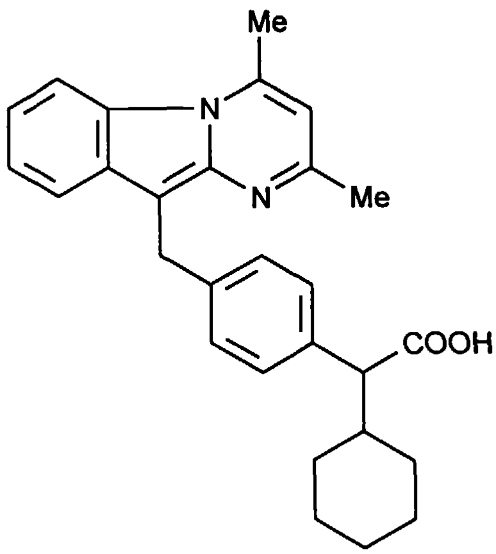

4,16 g (9,45 mmol) der Verbindung aus Beispiel CV werden 72 h in 132 ml 1 M wäßriger Natronlauge und 200 ml 1,4-Dioxan bei 60°C umgesetzt. Die Reaktionsmischung wird auf ca. 150 ml eingedampft, mit 400 ml Wasser versetzt und bei 0°C mit 2 M wäßriger Salzsäure auf einen pH-Wert von 2,5 gestellt. Der dabei anfallende Niederschlag wird abgesaugt, mit Wasser gewaschen, scharf abgesaugt und im Vakuum über Phosphorpentoxid getrocket.

Ausbeute: 3,5 g
$R_f$ = 0,20 (B)

Die racemischen Verbindungen der Tabelle XIII werden analog der Vorschrift von Beispiel CXLVIII hergestellt:

## Tabelle XIII

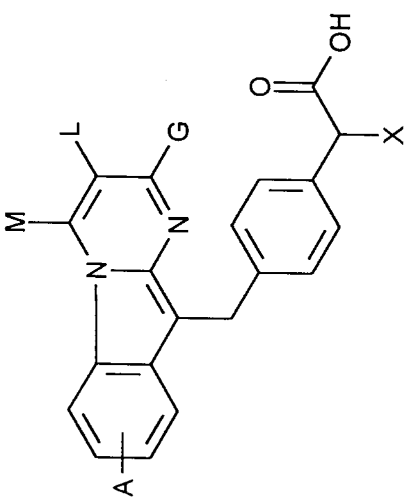

| Bsp.-Nr. | A | G | L | M | X | a) Fp. (°C)<br>b) $R_f$ (Solvens) | Spektren | Ausgangsmaterial aus Bsp.-Nr. |
|---|---|---|---|---|---|---|---|---|
| CIL | H | Me | H | Me | 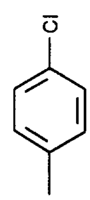 | b) 0,27 (I) | | CIX |

Die Verbindungen der Tabelle XIV werden analog der Vorschrift von Beispiel XCII hergestellt:

**Tabelle XIV:**

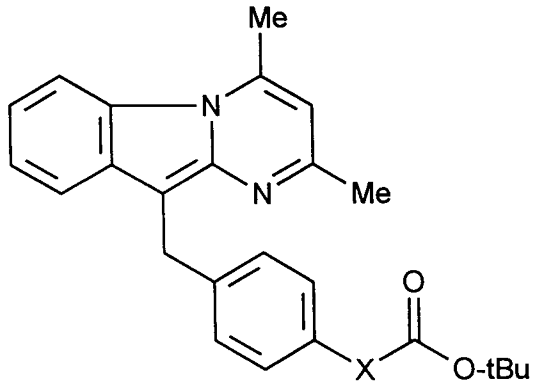

| **Bsp.-Nr.** | **-X-** | **a) Fp. (°C)**<br>**b) $R_f$ (Solvens)** | **Spektren** | **Ausgangs-material aus Bsp.-Nr.** |
|---|---|---|---|---|
| CL | 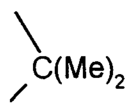 | b) 0,42 (G) | | LXXX und LV |
| CLI | 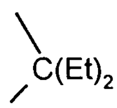 | b) 0,13 (A) | | LXXX und LVI |
| CLII | 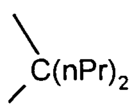 | b) 0,34 (C) | | LXXX und LVII |
| CLIII | 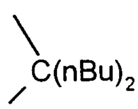 | b) 0,36 (C) | | LXXX und LVIII |
| CLIV | 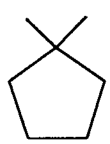 | b) 0,75 (J) | | LXXX und LIX |
| CLV | 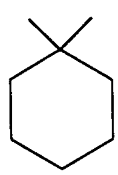 | b) 0,56 (J) | | LXXX und LX |

## Tabelle XIV - Fortsetzung

| Bsp.-Nr. | -X- | a) Fp. (°C) b) $R_f$ (Solvens) | Spektren | Ausgangs-material aus Bsp.-Nr. |
|---|---|---|---|---|
| CLVI | 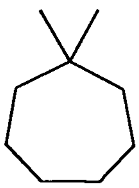 | b) 0,37 (K) | | LXXX und LXI |

Die Verbindungen der Tabelle XV werden analog der Vorschrift von Beispiel CXXI hergestellt:

## Tabelle XV:

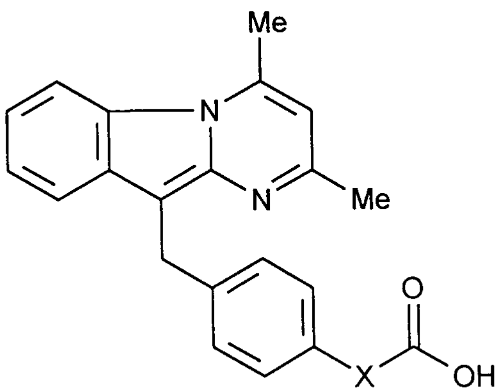

| Bsp.-Nr. | -X- | a) Fp. (°C) b) $R_f$ (Solvens) | Spektren | Ausgangs-material aus Bsp.-Nr. |
|---|---|---|---|---|
| CLVII | 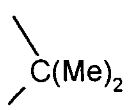 | b) 0,11 (B) | | CL |
| CLVIII | 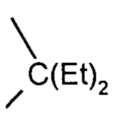 | | MS (CI, $NH_3$): 401 ($[M+H]^+$, 100%) | CLI |
| CLIX | 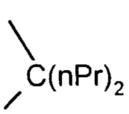 | b) 0,20 (B) | | CLII |
| CLX | 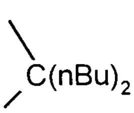 | b) 0,25 (B) | | CLIII |

**Tabelle XV - Fortsetzung**

| Bsp.-Nr. | -X- | a) Fp. (°C) b) $R_f$ (Solvens) | Spektren | Ausgangs-material aus Bsp.-Nr. |
|---|---|---|---|---|
| CLXI | 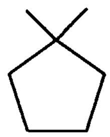 | | MS (FAB): 399 ($[M+H]^+$, 15%) | CLIV |
| CLXII | 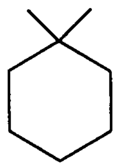 | | MS (FAB): 412 ($M^+$, 100%) | CLV |
| CLXIII | 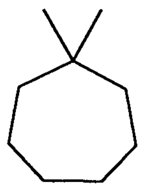 | | MS (CI, $NH_3$): 427 ($[M+H]^+$, 100%) | CLVI |

**Beispiel CLXIV**

2(R/S)-2-Cyclopentyl-2-(3-tolyl)-essigsäuremethylester

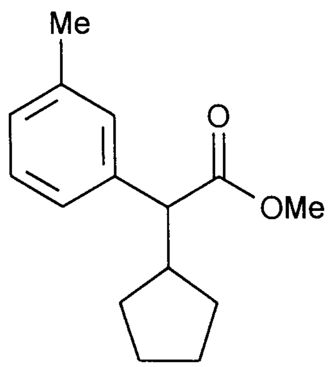

Die Titelverbindung wird analog der Vorschrift des Beispiels IV aus 2-(3-Tolyl)-essigsäuremethylester hergestellt.

$R_f$ = 0,56 (P)

**Beispiel CLXV**

2(R/S)-2-(3-Brommethyl-phenyl)-2-cyclopentyl-essigsäuremethylester

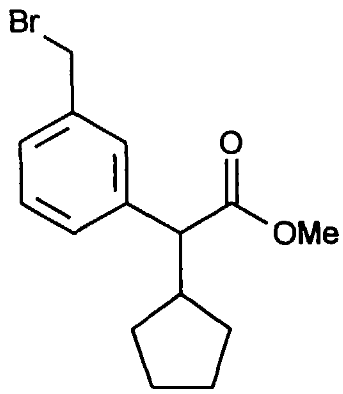

Die Titelverbindung wird analog der Vorschrift für Beispiel XXXII aus der Verbindung des Beispiels CLXIV hergestellt.

$R_f$ = 0,40 (P)

**Beispiel CLXVI**

2(R/S)-2-Cyclopentyl-2-(4-methylphenyl)-essigsäure

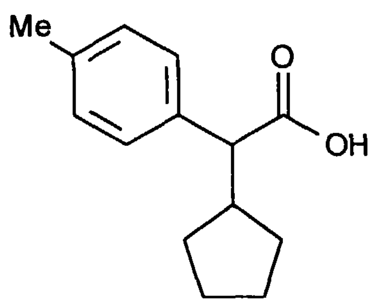

In einem 40 l-Rührwerkkessel mit angeschlossenem Waschturm werden 2,0 kg (7,2 mol) der Verbindung aus Beispiel IV in 4 l Dioxan gelöst. Nach Zugabe von 4,5 l konzentrierter Salzsäure wird bis zum vollständigen Umsatz (3h) bei 50°C gerührt. Das Reaktionsgemisch wird mit Eis versetzt und mit konzentrierter Natronlauge auf pH = 12 eingestellt. Nach Zugabe von Wasser bis zur vollständigen Auflösung der Feststoffe wird mit Essigester gewaschen, die organische Phase wird mit verdünnter Natronlauge gewaschen und die vereinigten wäßrigen Phasen unter Kühlung mit konzentrierter Salzsäure auf pH = 1 eingestellt. Es wird zweimal mit Essigester gewaschen, über Natriumsulfat getrocknet und eingeengt.

Ausbeute: 1,27 kg; 81% d.Th.
Schmpkt.: 92°C
$R_f$ = 0,20 (Petrolether : Essigester = 4:1)
$^1$H-NMR ($CDCl_3$, 200 MHz, TMS): δ = 0,98 (m, 1H); 1,20 - 1,71 (m, 6H); 1,82 - 2,05 (m, 1H); 2,31 (s, 3H); 2,52 (m, 1H); 3,21 (d, 1H); 7,10 (m, 2H); 7,21 m, 2H); 11,90 (br, s, 1H) ppm.

**Beispiel CLXVII**

(S)-(+)-2-Cyclopentyl-2-(4-methylphenyl)-essigsäure

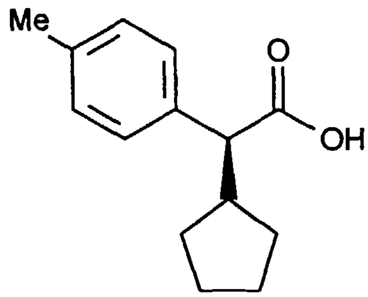

Zu einer Suspension von 560 g (2,57 mol) der Verbindung aus Beispiel CLXVI in 4,8 l Wasser werden unter Rühren 2,4 l THF und 129,7 g (1,28 mol) Triethylamin gegeben. Die entstehende Lösung wird auf 60°C erwärmt, es werden 155,4 g (1,28 mol) (S)-(-)-Phenethylamin zugegeben und die anfallende Suspension 2 h bei 60°C gerührt. Das Reaktionsgemisch wird auf 20°C gekühlt, der Niederschlag wird abgesaugt, mit 2,4 l Wasser / THF (2:1) gewaschen und im Vakuum getrocknet.

Ausbeute: 360 g Phenethylammoniumsalz; 41,3% d.Th. bezogen auf Racemat
Bsp.-Nr. CLXVI

In 3 l Wasser werden 745 g (2,2 mol) Phenethylammoniumsalz suspendiert, mit verdünnter Salzsäure (1:1) angesäuert (pH = 1) und 30 Minuten gerührt. Die ölige Suspension wird dreimal mit je 1 l Dichlormethan gewaschen, die vereinigten organischen Phasen mit Wasser gewaschen, über Natriumsulfat getrocknet und eingeengt, wobei der Rückstand kristallisiert.

Ausbeute: 475 g; 37,3% d.Th. bezogen auf Racemat Bsp.-Nr. CLXVI ee: 96,3% (HPLC)
Schmpkt.: 66°C

Durch Kristallisation des Phenethylammoniumsalzes aus THF und Freisetzung von Beispiel Nr. CLXVII, wie oben beschrieben wird das Reinenantiomer erhalten: ee: >99,5% (HPLC)

Drehwert: $[\alpha]^{20}_{D}$= +59,55 (Ethanol / c = 0,85)

Die HPLC-Methode zur Bestimmugn des ee.Wertes ist folgende (die racemische Verbindung aus Beispiel CLXVI dient als Vergleich):

| | |
|---|---|
| Säule: | Chiracel OJ (Daicel) |
| Partikelgröße: | 10 µ |
| Packing: | 250 x 2 mm (Fa. Grom) |
| Mobile Phase: | n-Heptan: 2-Propanol = 97:3 |
| Fluß: | 0,2 ml/min |
| Eingangsdruck: | 22 bar |

**Beispiel CLXVIII**

(S)-(+)-2-Cyclopentyl-2-(4-methylphenyl)essigsäure-tert.butylester

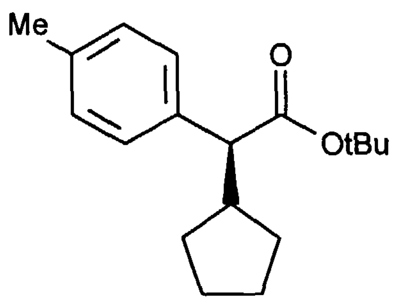

Zu einer Lösung von 465 g (2,13 mol) der Verbindung aus Beispiel CLXVII in 1,4 l Dichlormethan werden 6 ml konzentrierte Schwefelsäure gegeben, wobei sich eine Temperatur von ca. 10°C einstellt. In ein Dewargefäß werden 550 ml (5 mol) Isobuten einkondensiert und in einer Portion zur Eduktlösung gegeben. Das Reaktionsgemisch wird über Nacht gerührt. Zur Vervollständigung des Umsatzes werden nochmals 6 ml konzentrierte Schwefelsäure und 500 ml Isobuten zugegeben und über Nacht gerührt. Nach Zugabe von 40 g Kaliumcarbonat wird 3 h gerührt, und darauf 2 l Wasser zugegeben, wobei es anfangs zu einer starken Gasentwicklung kommt. Es wird dreimal mit je 2 l Dichlormethan gewaschen, die vereinigten organischen Phasen werden mit 5 l Natriumchloridlösung gewaschen, über Natriumsulfat getrocknet und zu einem Öl, das langsam durchkristallisiert, eingeengt.

Ausbeute: 480 g; 82% d.Th.
Schmpkt.: 45°C
$R_f$ = 0,90 (Toluol : Essigester = 8:2)

**Beispiel CLXIX**

(S)-(+)-2-(4-Brommethylphenyl)-2-cyclopentyl-essigsäure-tert.butylester

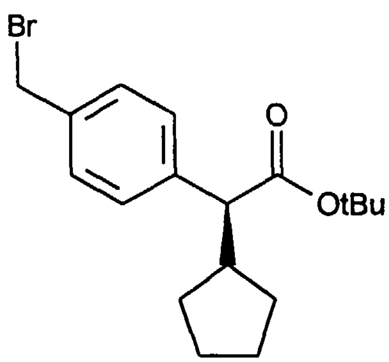

In einem 10 l-Kolben weden 480 g (1,75 mol) der Verbindung aus Beispiel CLXVIII in 3,4 l Tetrachlormethan unter Rückfluß gelöst und mit 70 g Gesamtmenge von 311 g (1,75 mol) NBS sowie 14 g (0,085 mol) AIBN versetzt. Die Reaktion setzt nach ca 1 h Refluxieren ein; nach Abklingen wird weiteres NBS in 50 g Portionen zugegeben. Nach 5 h Refluxieren und anschließendem Stehen bei Raumtemperatur über Nacht wird zur Aufarbeitung auf 0°C gekühlt, das Succinimid abgesaugt und mit 600 ml Tetrachlormethan nachgewaschen. Die vereinigten Filtrate werden eingeengt und Restlösemittel bis zur Gewichtskonstanz im Vakuum entfernt.

Rohausbeute: 570 g; ca. 100% d.Th.
HPLC: 68,8% (15,5% Edukt, 10,1% Dibromverbindung)

Der Reinstoff wird durch Säulenchromatographie erhalten

$R_f$ = 0,42 (Q)
$^1$H-NMR ($CDCl_3$, 200 MHz, TMS): δ = 0,98 (m, 1H); 1,22 - 1,71 (m, 6H); 1,40 (s, 9H); 1,90 (m, 1H); 2,47 (m, 1H); 3,16 (d, 1H); 4,49 (s, 2H); 7,32 (m, 4H) ppm.

**Beispiel CLXX**

2-(4-Tolyl)-essigsäure-(L)-menthylester

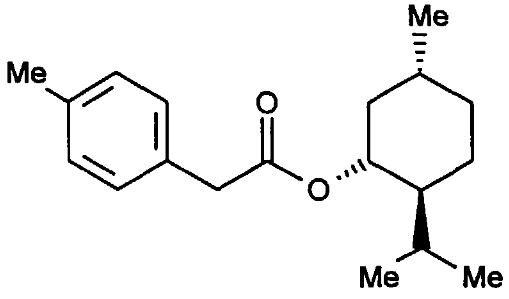

3,15 kg p-Tolylessigsäure und 9,45 l Toluol werden vorgelegt. Unter Rühren und Kühlen werden 3,115 kg L-Menthol und 21,4 ml Methansulfonsäure zugegeben. Anschließend wird auf Rückflußtemperatur erhitzt und innerhalb von 16 bis 20 Stunden über einen Wasserabscheider die entsprechende Menge Wasser abgetrennt. Nach Abkühlen auf Raumtemperatur wird einmal mit 4,41 l gesättigter Natriumhydrogencarbonat-Lösung und zweimal mit je 4,41 l Wasser ausgerührt. Die organische Phase wird vom Lösemittel befreit und ergibt 5,725 kg gewünschte Verbindung (GC 99,9%, Retentionszeit 19,49 min).

$^1$H-NMR ($CDCl_3$, ppm): 7,05 - 7,15 (4H, m); 4,55 (1H, txd); 3,5 (2H, s); 2,8 (3H, s); 0,65 (3H, s).

**Beispiel CLXXI**

2-(S)-2-Cyclopentyl-2-(4-tolyl)-essigsäure-(L)-menthylester

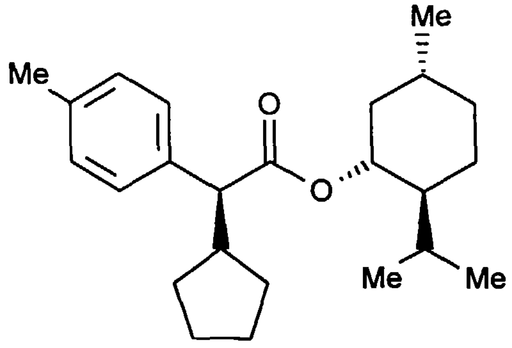

1,575 kg Kalium-tert.butanolat werden bei Raumtemperatur in 3,75 l DMF gelöst. Man kühlt auf 10°C ab und läßt innerhalb von 45 Minuten bei dieser Temperatur 2,678 kg der Verbindung aus Beispiel CLXX zulaufen und spült mit 0,375 l DMF nach. Innerhalb von 1 bis 2 Stunden werden nun unter voller Kühlung 1,658 kg Cyclopentylbromid zugepumpt. Die Suspension wird ohne Kühlung noch eine Stunde nachgerührt und dann auf -7°C abgekühlt. Bei Erreichen von -10°C wird mit dem richtigen Diastereomeren angeimpft und dann weiter auf -7°C gekühlt. Nach Erreichen von -7°C wird 3 bis 4 Stunden bei dieser Temperatur nachgerührt. Die Aufarbeitung erfolgt durch Einbringen der Reaktionssuspension in ein Gemisch aus 1,5 kg Eis und 6 kg Wasser. Der Ansatz wird danach bei 0 bis 2°C über Nacht gerührt.

Die Aufarbeitung erfolgt durch Absaugen der Suspension und Waschen der Kristalle mit insgesamt 2,5 l Wasser. Die Kristalle werden bei 45°C im Vakuumtrockenschrank getrocknet. Es werden 3,289 kg eine 85 zu 15 Diastereomerengemisches erhalten.

4,345 kg einer wie oben beschriebenen, hergestellten Mischung, werden in 21,75 l bei 30 bis 35°C gelöst. Nach Animpfen mit dem richtigen Diastereomeren und Abkühlen auf Raumtemperatur wird über Nacht gerührt und am nächsten Morgen auf 0 bis 5°C gekühlt. Nach 1 bis 2 Stunden bei dieser Temperatur werden die Kristalle abgesaugt, getrocknet oder erneut umkristallisiert. Durch ein bis zweimaliges Wiederholen der Methanolkristallisation kann Material mit einer Diastereomerenreinheit von ≥ 99,5% hergestellt werden (GC-Retentionszeit 22,61 min).

Die Ausbeute an diastereomerenreiner Titelverbindung liegt bei 65-70% über die Stufen Cyclopentylierung und Reinkristallisation und kann durch Rekristallisation bzw. durch Epimerisierung der Mutterlaugen mit Kalium-tert.butanolat in DMF und erneute Kristallisation des rohen Diastereomerengemisches auf 75-80% gesteigert werden.

$^{13}C$-NMR ($CDCl_3$, CH-Signale, ppm) 128.90; 128.92; 73.96; 57.85; 46.92; 43.13; 31.28; 25.96 ppm.

**Beispiel CLXXII**

2-(S)-2-(4-Brommethyl-phenyl)-2-cyclopentyl-essigsäure-(L)-menthylester

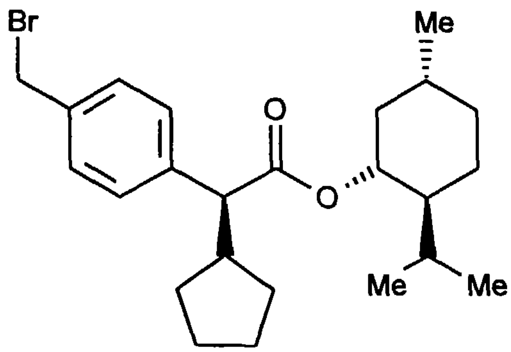

1,40 kg der Verbindung aus Beispiel CLXXI werden in 13,74 l Chlorbenzol auf 80°C erwärmt. Danach gibt man 0,618 kg 1,3-Dibrom-5,5-dimethylhydantoin hinzu und erwärmt weiter auf 85°C. Bei dieser Temperatur wird nun zum Starten der Reaktion 20,4 g AIBN gegeben. Die Temperatur steigt nach Beginn der Reaktion auf 90 bis 105°C, sinkt dann aber wieder auf etwa 85°C ab. Insgesamt wird 2 Stunden nachreagiert. Danach wird der Kesselinhalt auf Raumtemperatur abgekühlt und eine Stunde nachgerührt. Es wird von den ausgefallenen Kristallen abgesaugt und das Filtrat vom Lösemittel befreit. Das zurückbleibende Öl ist nach HPLC-Analyse (Retentionszeit 14,68 min.) 61,2%ig. Es werden 1,69 kg erhalten. Das Gemisch kann roh in die folgenden Alkylierungen eingesetzt werden. Chromatographie und nachfolgende Kristallisation liefern ein weißes Pulver vom Schmelzpunkt 57-58°C, mit korrekter CH-Analyse.

$^{1}H$-NMR ($CDCl_3$, ppm): 7,3 (4H, s); 4,65 (1H, txd); 4,45 (2H, s); 3,35 (1H, d); 0,65 (3H, d).

**Beispiel CLXXIII**

2-(R/S)-2-Phenyl-2-(4-methylphenyl)-essigsäuremethylester

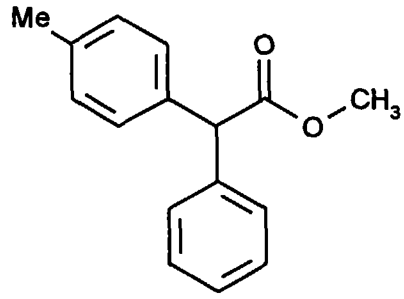

21,0 g (100 mmol) 2-Phenyl-1-(4-methylphenyl)-1-oxoethan und 38,8 g (120 mmol) Iodbenzoldiacetat werden in 300 ml Orthoameisensäuretrimethylester gelöst. Zu dieser Lösung werden 19,6 g konzentrierte Schwefelsäure gegeben, und die Lösung wird für 6 h bei 60°C gerührt. Die Lösung wird auf Raumtemperatur abgekühlt, mit Wasser vedünnt, und mit Diethylether extrahiert. Die vereinigten organischen Phasen werden über Natriumsulfat getrocknet und einrotiert. Der Rückstand wird säulenchromatographisch gereinigt.

Ausbeute: 13,1 g (55%)
$R_f$ = 0,33 (Q)
MS (FAB): 241 (25%), 181 (100%).
$^1$H-NMR (200 MHz, $CDCl_3$, TMS): δ = 7,3 - 7,10 (m, 9H); 4,99 (s, 1H); 3,73 (s, 3H); 2,31 (s, 3H) ppm.

**Beispiel CLXXIV**

2-(R/S)-2-(4-Chlorphenyl)-2-(4-tolyl)-essigsäure-methylester

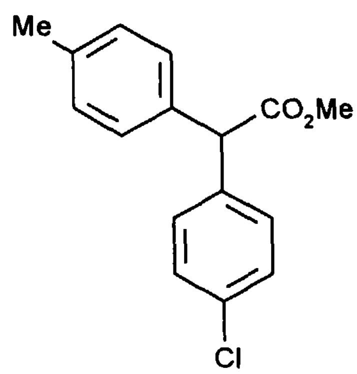

In Analogie zur Vorschrift des Beispiels CLXXIII wird die Titelverbindung hergestellt. $R_f$ = 0.41 (Q)
Die racemischen Verbindungen der Tabelle XVI werden analog der Vorschrift des Beispiels XXXII hergestellt:

**Tabelle XVI**

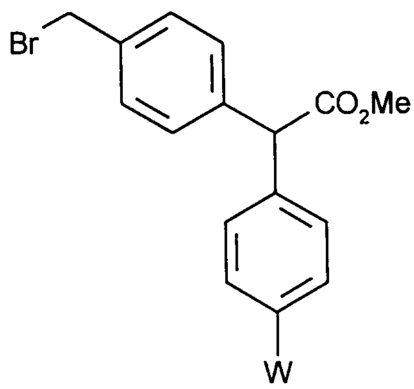

| Bsp.-Nr. | W | a) Fp. (°C) b) $R_f$ (Solvens) | Spektren | Ausgangs-material aus Bsp.-Nr. |
|---|---|---|---|---|
| CLXXV | H | b) 0,74 (G) | | CLXXIII |
| CLXXVI | Cl | b) 0,28 (Q) | | CLXXIV |

**Herstellungsbeispiele**

**Beispiel 1**

N-{2(R)- und 2(S)-2-Cyclopentyl-2-[4-(2,4-dimethyl-pyrimido[1,2-a]indol-9-yl-methyl)phenyl]-acetyl}(R)phenylglycinol

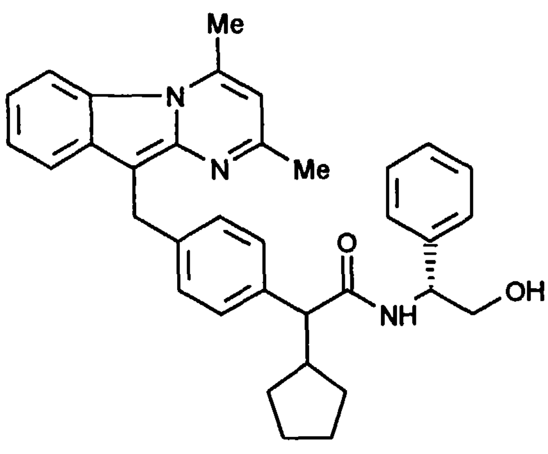

1,88 g (4,56 mmol) der Verbindung aus Beispiel CXXII werden in 20 ml Dichlormethan mit 0,63 g (4,56 mmol) (R)-Phenylglycinol, 0,68 g (5,00 mmol) 1-Hydroxy-1H-benzotriazol (HOBI), 1,01 g (5,20 mmol) N'-(3-Dimethylaminopropyl)-N-ethylcarbodiimid (CDI) Hydrochlorid und 1,27 g (9,12 mmol) Triethylamin 20 h bei ca. 20°C umsetzt. Darauf wird die Reaktionslösung zuerst mit einem Puffer von pH = 2 (Merck), dann mit Magnesiumsulfat getrocknet und eingedampft. Das rohe Diastereomerengemisch wird an Kieselgel 60 (Merck / 40-63 µm / Dichlormethan: Ethanol = 20:1) aufgereinigt.

Ausbeute: 2,20 g (4,14 mmol) 91% d.Th.

Die Trennung der Isomeren (Beispiele 2 und 3) gelingt chromatographisch.
Es werden 452 mg der unter Beispiel 1 beschriebenen Ausbeute in 10 ml Acetonitril , 13 ml Methanol und 2 ml Wasser gelöst und die erhaltene Lösung in mehreren Injektionsschritten auf die HPLC-Säule aufgegeben.

| | |
|---|---|
| Stationäre Phase: | Kromasil 100 C18 5 µm |
| Temperatur: | 50°C |
| Fluß: | 20 ml/min |
| Laufmittel: | Acetonitril : Wasser : Methanol = 37,5 : 25 : 37,5 |

Die Eluate werden nach UV-Detektion (230 nm) gesammelt und zuerst im Vakuum eingedampft, dann zur Entfernung des Restlösemittels gefriergetrocknet.

Diastereomer A (Beispiel 2): 176 mg
Diastereomer B (Beispiel 3): 160 mg

**Beispiel 2**

N-{2(S)-2-Cyclopentyl-2-[4-(2,4-dimethyl-pyrimido[1,2-a]indol-9-yl-methyl)phenyl]-aceryl}(R)phenylglycinol

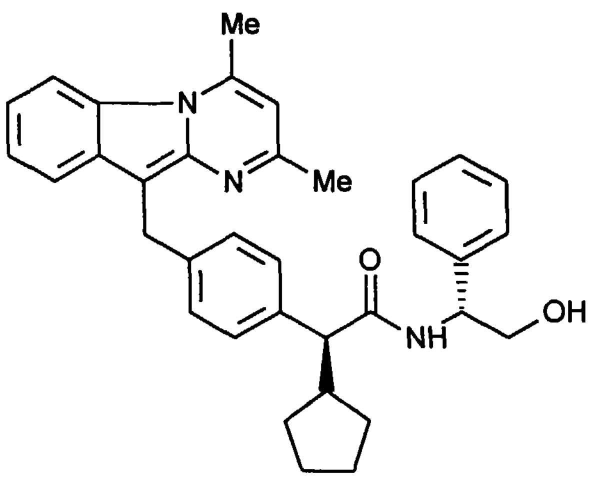

DC: $R_f$ =0,19 (Petrolether : Essigsäureethylester = 1:1) = E
Schmp.: 208°C (unkorrigiert).
$^1$H-NMR ($CDCl_3$, 300 MHz, TMS): δ = 0,97 (m, 1H); 1,26 (m, 1H); 1,38 - 2,02 (m, 6H); 2,28 (m, 1H); 2,51 (s, 3H); 2,55 (m, 1H); 2,94 (s, 3H); 3,03 (d, 1H); 3,77 - 3,89 (m, 2H); 4,48 (s, 2H); 4,94 (m, 1H); 5,99 (d, 1H); 6,17 (s, 1H); 6,96 - 7,00 (m, 2H); 7,10 - 7,34 (m, 9H); 7,67 (m, 1H); 8,07 (m, 1H) ppm.

**Beispiel 3**

N-{2(R)-2-Cyclopentyl-2-[4-(2,4-dimethyl-pyrimido[1,2-a]indol-9-yl-methyl)phenyl]-acetyl}(R)phenylglycinol

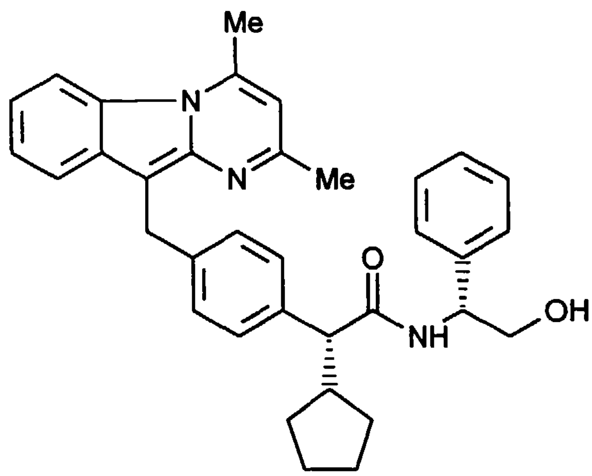

DC: $R_f$ = 0,19 (Petrolether : Essigsäureethylester = 1:1) = E
Schmp.: 191°C (unkorrigiert)
$^1$H-NMR ($CDCl_3$, 200 MHz, TMS): δ = 0,97 (m, 1H); 1,22 (m, 1H); 1,36 - 1,68 (m, 6H); 1,86 (m, 1H); 2,49 (m, 1H); 2,52 (s, 3H); 2,94 (s, 3H); 3,01 (d, 1H); 3,66 - 3,81 (m, 2H); 4,48 (s, 2H); 4,93 (m, 1H); 6,07 (d, 1H); 6,18 (s, 1H); 7,13 - 7,37 (m, 11H); 7,66 (m, 1H); 8,06 (m, 1H) ppm.

Die absolute Konfiguration der enantiomerenreinen Carbonsäuren 2-(S)- und 2-(R)-2-{4-(Chinolin-2-yl-methoxy)-phenyl}-2-cyclopentyl-essigsäure [vgl. EP 509 359] sind bekannt, so daß die absoluten Konfigurationen der daraus analog der Vorschrift zu den Beispielen 1 und 2 hergestellten Amiden Bsp.-Nr. C1 und Bsp.-Nr. C2 abgeleitet werden können. Die $^1$H-NMR-Spektren der beiden diastereomeren Produkte (200 MHz, $d_6$-DMSO, TMS für Beispiel-Nr. C1 und 250 MHz, $d_6$-DMSO, TMS für Beispiel-Nr. C2 / Abbildung 1) weisen im Aromatenbereich signifikante Unterschiede auf: Die H-Signale des Phenylrestes von Beispiel-Nr. C1 bei ca. 7.1 ppm (3H) und 7.3 ppm (2H), die H-Signale von Beispiel-Nr. C2 bei ca. 7.3 ppm (5H). Dieser Befund ist auf die Verbindungen der Beispiele 2 und 3 (Abbildung 2) sowie auf andere Derivate diesen Typs übertragen und die angegebenen absoluten und relativen Konfigurationen so ermittelt worden.

Die Verbindungen der Tabelle 1 werden analog der Vorschrift von Beispiel 1, 2 und 3 hergestellt:

**Tabelle 1:**

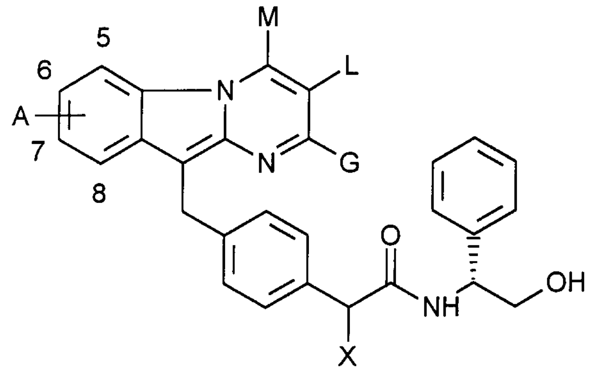

| Bsp.-Nr. | Isomer | A | G | L | M | X | a) Fp. (°C) b) $R_f$ (Solvens) | Spektren | Ausgangs-material aus Bsp.-Nr. |
|---|---|---|---|---|---|---|---|---|---|
| 4 | / | H | Me | H | Me | H | | MS (FAB): 464 ($[M+H]^+$, 20%) | CXXII |
| 5 | 2 dia | H | Me | H | Me | Me | b) 0,28/0,33 (D) | | CXXIII |
| 6 | dia A | H | Me | H | Me | Me | b) 0,28 (D) | | CXXIII |
| 7 | dia B | H | Me | H | Me | Me | b) 0,33 (D) | | CXXIII |
| 8 | 2 dia | H | Me | H | Me | Et | b) 0,28/0,31 (D) | | CXXIV |
| 9 | dia A | H | Me | H | Me | Et | b) 0,28 (D) | | CXXIV |
| 10 | dia B | H | Me | H | Me | Et | b) 0,31 (D) | | CXXIV |
| 11 | 2 dia | H | Me | H | Me | nPr | b) 0,46/0,63 (D) | | CXXV |
| 12 | dia A | H | Me | H | Me | nPr | b) 0,63 (D) | | CXXV |
| 13 | dia B | H | Me | H | Me | nPr | b) 0,46 (D) | | CXXV |
| 14 | 2 dia | H | Me | H | Me | nBu | b) 0,44 (D) | | CXXVI |

Tabelle 1 - Fortsetzung

| Bsp.-Nr. | Isomer | A | G | L | M | X | a) Fp. (°C)<br>b) $R_f$ (Solvens) | Spektren | Ausgangs-material aus Bsp.-Nr. |
|---|---|---|---|---|---|---|---|---|---|
| 15 | dia A | H | Me | H | Me | nBu | b) 0,44 (D) | | CXXVI |
| 16 | dia B | H | Me | H | Me | nBu | b) 0,44 (D) | | CXXVI |
| 17 | 2 dia | H | Me | H | Me | iBu | b) 0,42 (D) | | CXXVII |
| 18 | dia A | H | Me | H | Me | iBu | b) 0,42 (D) | | CXXVII |
| 19 | dia B | H | Me | H | Me | iBu | b) 0,42 (D) | | CXXVII |
| 20 | 2 dia | H | Me | H | Me | nPent | a) 149°C | | CXXVIII |
| 21 | dia A | H | Me | H | Me | nPent | | MS (FAB) 533 ($M^+$, 100%) | CXXVIII |
| 22 | dia B | H | Me | H | Me | nPent | a) 140°C | | CXXVIII |
| 23 | 2 dia | H | Me | H | Me | iPent | a) 174°C | | CXXIX |
| 24 | dia A | H | Me | H | Me | iPent | a) 164°C | | CXXIX |
| 25 | dia B | H | Me | H | Me | iPent | a) 204°C | | CXXIX |
| 26 | 2 dia | H | Me | H | Me | $-CH(Et)_2$ | a) 110°C | | CXXX |
| 27 | dia A | H | Me | H | Me | $-CH(Et)_2$ | a) 112°C | | CXXX |
| 28 | dia B | H | Me | H | Me | $-CH(Et)_2$ | a) 193°C | | CXXX |
| 29 | 2 dia | H | Me | H | Me | nHex | b) 0,63/0,58 (D) | | CXXXI |
| 30 | dia A | H | Me | H | Me | nHex | b) 0,63 (D) | | CXXXI |
| 31 | dia B | H | Me | H | Me | nHex | b) 0,58 (D) | | CXXXI |
| 32 | 2 dia | H | Me | H | Me | $-CH_2CH(Et)_2$ | a) 179°C | | CXXXII |

Tabelle 1 - Fortsetzung

| Bsp.-Nr. | Isomer | A | G | L | M | X | a) Fp. (°C) b) $R_f$ (Solvens) | Spektren | Ausgangs-material aus Bsp.-Nr. |
|---|---|---|---|---|---|---|---|---|---|
| 33 | dia A | H | Me | H | Me | $-CH_2CH(Et)_2$ | | MS (FAB) 547 ($M^+$, 100%) | CXXXII |
| 34 | dia B | H | Me | H | Me | $-CH_2CH(Et)_2$ | a) 181°C | | CXXXII |
| 35 | 2 dia | H | Me | H | Me | cHex | b) 0,31 (E) | | CXXXIII |
| 36 | dia A | H | Me | H | Me | cHex | b) 0,31 (E) | | CXXXIII |
| 37 | dia B | H | Me | H | Me | cHex | b) 0,31 (E) | | CXXXIII |
| 38 | 2 dia | H | Me | H | Me | cHept | b) 0,42 (D) | | CXXXIV |
| 39 | dia A | H | Me | H | Me | cHept | b) 0,42 (D) | | CXXXIV |
| 40 | dia B | H | Me | H | Me | cHept | b) 0,42 (D) | | CXXXIV |
| 41 | 2 dia | H | Me | H | Me | cOct | b) 0,35/0,40 (D) | | CXXXV |
| 42 | dia A | H | Me | H | Me | cOct | b) 0,40 (D) | | CXXXV |
| 43 | dia B | H | Me | H | Me | cOct | b) 0,35 (D) | | CXXXV |
| 44 | 2 dia | H | Me | H | Me | $-CH_2$-cPent | b) 0,32 (D) | | CXXXVI |
| 45 | dia A | H | Me | H | Me | $-CH_2$-cPent | b) 0,32 (D) | | CXXXVI |
| 46 | dia B | H | Me | H | Me | $-CH_2$-cPent | b) 0,32 (D) | | CXXXVI |
| 47 | 2 dia | H | Me | H | Me | 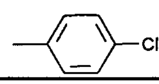 | a) 122°C | | CIL |
| 48 | dia A | H | Me | H | Me | 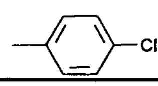 | a) 226°C | | CIL |

**Tabelle 1 - Fortsetzung**

| Bsp.-Nr. | Isomer | A | G | L | M | X | a) Fp. (°C)<br>b) $R_f$ (Solvens) | Spektren | Ausgangs-material aus Bsp.-Nr. |
|---|---|---|---|---|---|---|---|---|---|
| 49 | dia B | H | Me | H | Me | 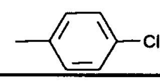 | a) 216°C | | CIL |
| 50 | 2 dia | H | Me | H | Et | cPent | a) 202°C | | CXXXVII |
| 51 | 2 dia | H | Et | H | Me | cPent | a) 172°C | | CXXXVIII |
| 52 | 2 dia | H | Et | H | Et | cPent | a) 190°C | | CXXXIX |
| 53 | 2 dia | H | Me | Me | Me | cPent | a) 215°C | | CXL |
| 54 | dia A | H | Me | Me | Me | cPent | a) 197°C | | CXL |
| 55 | dia B | H | Me | Me | Me | cPent | a) > 230°C | | CXL |
| 56 | 2 dia | H | Me | Et | Me | cPent | b) 0,13 (B) | | CXLV |
| 57 | 2 dia | H | Et | Me | Me | cPent | b) 0,33 (D) | | CXLVI |
| 58 | 2 dia | H | Me | Me | Et | cPent | b) 0,28 (D) | | CXLVII |
| 59 | 2 dia | 6-Cl | Me | H | Me | cPent | a) 160°C | | CXLI |
| 60 | 2 dia | 7-Cl | Me | H | Me | cPent | a) 158°C | | CXLII |
| 61 | 2 dia | 6-OMe | Me | H | Me | cPent | a) 144°C | | CXLIII |
| 62 | 2 dia | 7-OMe | Me | H | Me | cPent | a) 155°C | | CXLIV |

Die Verbindungen der Tabelle 2 werden analog der Vorschrift von Beispiel 1, 2 und 3 hergestellt:

**Tabelle 2:**

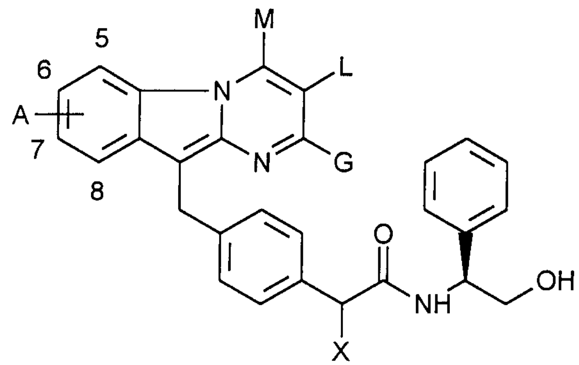

| Bsp.-Nr. | Isomer | A | G | L | M | X | a) Fp. (°C)<br>b) $R_f$ (Solvens) | Spektren | Ausgangsmaterial aus Bsp.-Nr. |
|---|---|---|---|---|---|---|---|---|---|
| 63 | 2 dia | H | Me | H | Me | cPent | b) 0,23 (F) | | CXXI |
| 64 | dia A | H | Me | H | Me | cPent | b) 0,23 (F) | | CXXI |
| 65 | dia B | H | Me | H | Me | cPent | b) 0,23 (F) | | CXXI |
| 66 | dia A | H | Me | Me | Me | cPent | a) 198°C | | CXL |
| 67 | dia B | H | Me | Me | Me | cPent | a) >230°C | | CXL |

Die Verbindungen der Tabelle 3 werden analog der Vorschrift des Beispiels 1 hergestellt:

**Tabelle 3:**

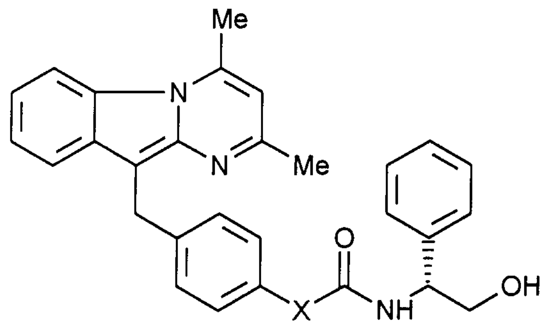

| Bsp.-Nr. | -X- | a) Fp. (°C)<br>b) $R_f$ (Solvens) | Spektren | Ausgangsmaterial aus Bsp.-Nr. |
|---|---|---|---|---|
| 68 | 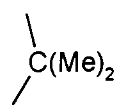 | b) 0,42 (F) | | CL VII |
| 69 | 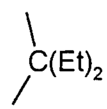 | a) 76°C | | CL VIII |
| 70 | 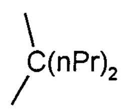 | b) 0,26 (F) | | CLIX |
| 71 | 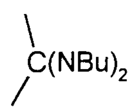 | b) 0,21 (F) | | CLX |

Tabelle 3 - Fortsetzung

| Bsp.-Nr. | -X- | a) Fp. (°C)<br>b) $R_f$ (Solvens) | Spektren | Ausgangsmaterial aus Bsp.-Nr. |
|---|---|---|---|---|
| 72 | 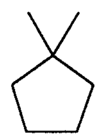 | a) 85°C | | CLXI |
| 73 | 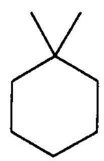 | a) 174°C | | CLXII |
| 74 | 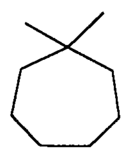 | a) 166°C | | CLXIII |

**Patentansprüche**

1. Pyrimido[1,2-a]indole der allgemeinen Formel (I)

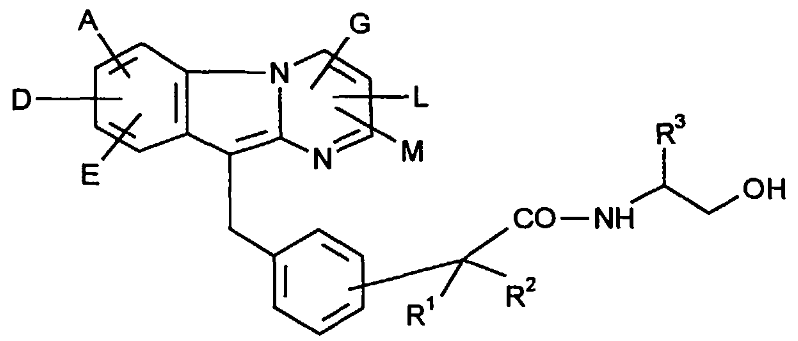

(I)

in welcher

A, D, E, G, L und M gleich oder verschieden sind und für Wasserstoff, Halogen, Trifluormethyl, Carboxy, Hydroxy, geradkettiges oder verzweigtes Alkoxy oder Alkoxycarbonyl mit jeweils bis zu 6 Kohlenstoffatomen oder geradkettiges oder verzweigtes Alkyl mit bis zu 6 Kohlenstoffatomen stehen, das seinerseits durch Hydroxy oder durch geradkettiges oder verzweigtes Alkoxy mit bis zu 4 Kohlenstoffatomen substituiert sein kann,

$R^1$ und $R^2$ gleich oder verschieden sind und für Wasserstoff, Cycloalkyl mit 3 bis 8 Kohlenstoffatomen oder geradkettiges oder verzweigtes Alkyl mit bis zu 10 Kohlenstoffatomen stehen, das gegebenenfalls durch Cycloalkyl mit 3 bis 6 Kohlenstoffatomen substituiert ist, oder für Phenyl stehen, das gegebenenfalls durch Halogen oder Trifluormethyl substituiert ist, oder

$R^1$ und $R^2$ gemeinsam mit dem Kohlenstoffatom einen 4-8 gliedrigen Cycloalkylring bilden

und

$R^3$ für Phenyl steht, das gegebenenfalls bis zu 3-fach gleich oder verschieden durch Nitro, Carboxy, Halogen, Cyano oder durch geradkettiges oder verzweigtes Alkenyl oder Alkoxycarbonyl mit jeweils bis zu 6 kohlenstoffatomen oder durch geradkettiges oder verzweigtes Alkyl mit bis zu 6 Kohlenstoffatomen substituiert ist, das gegebenenfalls durch Hydroxy, Carboxy oder durch geradkettiges oder verzweigtes Alkoxy oder Alkoxycarbonyl mit jeweils bis zu 6 Kohlenstoffatomen substituiert ist, und/oder gegebenenfalls durch eine Gruppe der Formel $-OR^4$ oder $-NR^5R^6$ substituiert ist, worin

$R^4$ Wasserstoff oder geradkettiges oder verzweigtes Alkyl oder Alkenyl mit jeweils bis zu 6 Kohlenstoffatomen bedeutet,

$R^5$ bzw. $R^6$ gleich oder verschieden sind und Phenyl, Wasserstoff oder geradkettiges oder verzweigtes Alkyl mit bis zu 6 Kohlenstoffatomen bedeuten, oder geradkettiges oder verzweigtes Acyl mit bis zu 8 Kohlenstoffatomen bedeuten, das gegebenenfalls durch eine Gruppe der Formel $-NR^7R^8$ substituiert ist, worin

$R^7$ und $R^8$ gleich oder verschieden sind und Wasserstoff oder geradkettiges oder verzweigtes Acyl mit bis zu 8 Kohlenstoffatomen bedeuten,

gegebenenfalls in einer isomeren Form und deren Salze.

2. Pyrimido[1,2-a]indole der Formel nach Anspruch 1, in welcher

A, D, E, G, L und M gleich oder verschieden sind und für Wasserstoff Fluor, Chlor, Brom, Trifluormethyl, Carboxy, Hydroxy, geradkettiges oder verzweigtes Alkoxy oder Alkoxycarbonyl mit jeweils bis zu 4 Kohlenstoffatomen oder geradkettiges oder verzweigtes Alkyl mit bis zu 4 Kohlenstoffatomen stehen, das seinerseits durch Hydroxy oder durch geradkettiges oder verzweigtes Alkoxy mit bis zu 3 Kohlenstoffatomen substituiert sein kann,

$R^1$ und $R^2$ gleich oder verschieden sind und für Wasserstoff, Cyclopropyl, Cyclobutyl, Cyclopentyl, Cyclohexyl, Cycloheptyl, Cyclooctyl oder für geradkettiges oder verzweigtes Alkyl mit bis zu 8 Kohlenstoffatomen stehen, das gegebenenfalls durch Cyclopropyl, Cyclopentyl oder Cyclohexyl substituiert ist, oder für Phenyl stehen, das gegebenenfalls durch Fluor, Chlor oder Brom substituiert ist, oder

$R^1$ und $R^2$ gemeinsam mit dem Kohlenstoffatom einen 4-7 gliedrigen Cycloalkylring bilden

und

$R^3$ für Phenyl steht, das gegebenenfalls bis zu 3-fach gleich oder verschieden durch Nitro, Carboxy, Fluor, Chlor, Brom, Cyano, durch geradkettiges oder verzweigtes Alkenyl oder Alkoxycarbonyl mit jeweils bis zu 4 Kohlenstoffatomen oder durch geradkettiges oder verzweigtes Alkyl mit bis zu 5 Kohlenstoffatomen substituiert ist, das gegebenenfalls durch Hydroxy, Carboxy oder durch geradkettiges oder verzweigtes Alkoxy oder Alkoxycarbonyl mit jeweils bis zu 5 Kohlenstoffatomen substituiert ist, und/oder gegebenenfalls durch eine Gruppe der Formel $-OR^4$ oder $-NR^5R^6$ substituiert ist, worin

$R^4$ Wasserstoff oder geradkettiges oder verzweigtes Alkyl oder Alkenyl mit jeweils bis zu 4 Kohlenstoffatomen bedeutet,

$R^5$ und $R^6$ gleich oder verschieden sind und Phenyl, Wasserstoff oder geradkettiges oder verzweigtes Alkyl mit bis zu 5 Kohlenstoffatomen bedeuten, oder geradkettiges oder verzweigtes Acyl mit bis zu 6 Kohlenstoffatomen bedeuten, das gegebenenfalls durch eine Gruppe der Formel $-NR^7R^8$ substituiert ist, worin

$R^7$ und $R^8$ gleich oder verschieden sind und Wasserstoff oder geradkettiges oder verzweigtes Acyl mit bis zu 6 Kohlenstoffatomen bedeuten,

gegebenenfalls in einer isomeren Form und deren Salze.

3. Pyrimido[1,2-a]indole der Formel nach Anspruch 1, in welcher

A, D, E, G, L und M gleich oder verschieden sind und für Wasserstoff, Fluor, Chlor, Brom, Trifluormethyl, Carboxy, Hydroxy, geradkettiges oder verzweigtes Alkoxy oder Alkoxycarbonyl mit jeweils bis zu 3 Kohlenstoffatomen oder für geradkettiges oder verzweigtes Alkyl mit bis zu 3 Kohlenstoffatomen stehen,

$R^1$ und $R^2$ gleich oder verschieden sind und für Wasserstoff, Cyclopropyl, Cyclobutyl, Cyclopentyl, Cyclohexyl, Cycloheptyl, Cyclooctyl oder für geradkettiges oder verzweigtes Alkyl mit bis zu 6 Kohlenstoffatomen stehen, das gegebenenfalls durch Cyclopentyl oder Cyclohexyl substituiert ist, oder für Phenyl stehen, das gegebenenfalls durch Fluor, Chlor oder Brom substituiert ist, oder

$R^1$ und $R^2$ gemeinsam mit dem Kohlenstoffatom einen 5-7 gliedrigen Cycloalkylring bilden

und

$R^3$ für Phenyl steht, das gegebenenfalls bis zu 3-fach gleich oder verschieden durch Hydroxy, Trifluormethyl,

Trifluormethoxy, Carboxy, oder durch geradkettiges oder verzweigtes Alkoxy, Alkyl oder Alkoxycarbonyl mit jeweils bis zu 3 Kohlenstoffatomen substituiert ist,

gegebenenfalls in einer isomeren Form und deren Salze.

**4.** Pyrimido[1,2-a]indole nach Anspruch 1 bis 3 als Arzneimittel.

**5.** Verfahren zur Herstellung von Pyrimido[1,2-a]indolen nach Anspruch 1 bis 3, dadurch gekennzeichnet, daß man racemische oder auch bereits enantiomerenreine Carbonsäuren oder deren aktivierte Derivate der allgemeinen Formel (II)

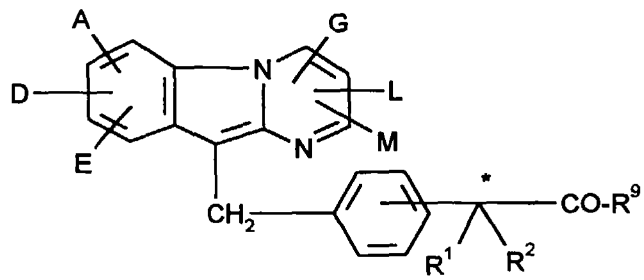

(II)

racemisch oder enantiomerenrein

in welcher

A, D, E, G, L, M, $R^1$ und $R^2$ die angegebene Bedeutung haben

und

$R^9$ für Hydroxy oder für einen aktivierenden Rest, vorzugsweise Chlor steht,

mit Phenylglycinolen der allgemeinen Formel (III)

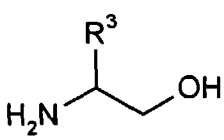

(III)

in welcher

$R^3$ die angegebene Bedeutung hat,

in inerten Lösemitteln, gegebenenfalls in Anwesenheit von Basen und/oder Hilfsstoffen amidiert.

**6.** Arzneimittel enthaltend mindestens ein Pyrimido[1,2-a]indol nach Anspruch 1 bis 3.

**7.** Arzneimittel nach Anspruch 6 zur Behandlung von Atherosklerose.

**8.** Carbonsäuren der allgemeinen Formel (II)

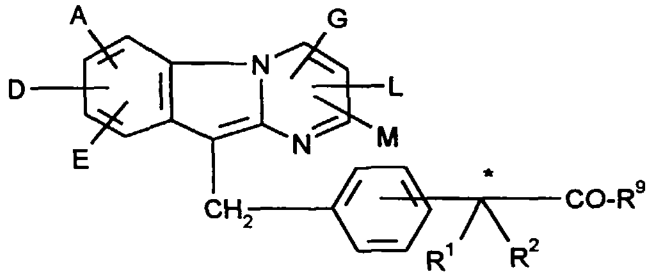

(II)

in welcher

A, D, E, G, L und M gleich oder verschieden sind und für Wasserstoff, Halogen, Trifluormethyl, Carboxy, Hydroxy, geradkettiges oder verzweigtes Alkoxy oder Alkoxycarbonyl mit jeweils bis zu 6 Kohlenstoffatomen oder geradkettiges oder verzweigtes Alkyl mit bis zu 6 Kohlenstoffatomen stehen, das seinerseits durch Hydroxy oder durch geradkettiges oder verzweigtes Alkoxy mit bis zu 4 Kohlenstoffatomen substituiert sein kann,

$R^1$ und $R^2$ gleich oder verschieden sind und für Wasserstoff, Cycloalkyl mit 3 bis 8 Kohlenstoffatomen oder geradkettiges oder verzweigtes Alkyl mit bis zu 10 Kohlenstoffatomen stehen, das gegebenenfalls durch Cycloalkyl mit 3 bis 6 Kohlenstoffatomen substituiert ist, oder für Phenyl stehen, das gegebenenfalls durch Halogen oder Trifluormethyl substituiert ist, oder

$R^1$ und $R^2$ gemeinsam mit dem Kohlenstoffatom einen 4-8 gliedrigen Cycloalkylring bilden

und

$R^9$ für Hydroxy oder für einen aktivierenden Rest steht,

und deren Salze.

**9.** Verfahren zur Herstellung von Carbonsäuren aus Verbindungen der allgemeinen Formel (IV), nach Anspruch 8 dadurch gekennzeichnet, daß man

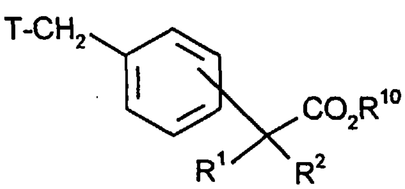

(IV)

in welcher

$R^1$ und $R^2$ die angegebene Bedeutung haben,

T für eine typische Abgangsgruppe wie beispielsweise Chlor, Brom, Jod, Tosylat oder Mesylat, vorzugsweise für Brom steht,

und

$R^{10}$ für ($C_1$-$C_4$)-Alkyl steht,

mit Verbindungen der allgemeinen Formel (V)

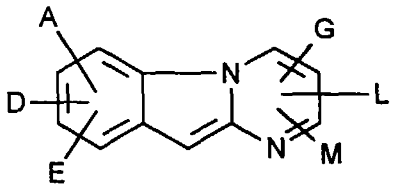

(V)

in welcher

A, D, E, G, L und M die angegebene Bedeutung haben,

die Verbindungen der allgemeinen Formel (VI)

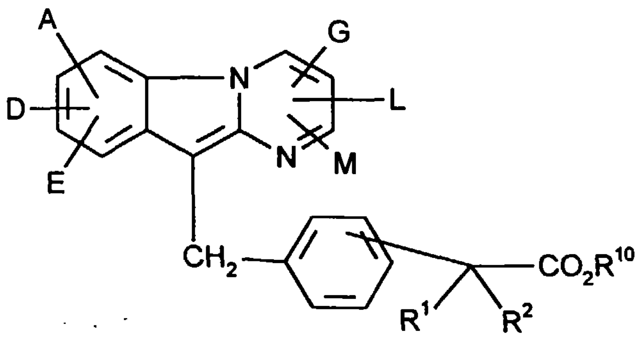

(VI)

in welcher

A, D, E, G, L, M, $R^1$, $R^2$ und $R^{10}$ die oben angegebene Bedeutung haben,

in inerten Lösemitteln, gegebenenfalls in Anwesenheit von Basen herstellt und anschließend die Ester nach üblichen Methoden verseift.

**10.** Verwendung von Pyrimido[1,2-a]indolen nach Anspruch 1 bis 3 zur Herstellung von Arzneimitteln.